# EUROPEAN PATENT SPECIFICATION

(11) **EP 4 395 855 B1**
(45) Date of publication and mention of the grant of the patent: **18.11.2026**
(21) Application number: 22769665.5
(22) Date of filing: 29.08.2022
(51) Int. Cl.: A61M 5/20, A61M 5/24, A61M 5/28, A61M 5/31, A61M 5/315

(54) **DEVICE FOR ADMINISTERING A PHARMACEUTICAL SUSPENSION**
VORRICHTUNG ZUR VERABREICHUNG EINER PHARMAZEUTISCHEN SUSPENSION
DISPOSITIF D'ADMINISTRATION D'UNE SUSPENSION PHARMACEUTIQUE

(30) Priority: 31.08.2021 EP 21194033; 26.11.2021 EP 21210767
(43) Date of publication of application: 10.07.2024
(73) Proprietor: Novo Nordisk A/S, 2880 Bagsvaerd (DK)
(72) Inventor: BENGTSSON, Henrik, 2880 Bagsværd (DK); DEHLI, Jeppe Ibsen, 2880 Bagsværd (DK); HELBO, Janus, 2880 Bagsværd (DK); EGENFELDT, Svend, 2880 Bagsværd (DK); WINDUM, Jesper Peter, 2880 Bagsværd (DK)
(86) International application number: PCT/EP2022/073887
(87) International publication number: WO 2023/031082

(56) References cited:
- US-A- 5 630 800
- US-A1- 2011 270 220
- US-A1- 2014 005 610
- US-A1- 2015 045 773
- US-A1- 2015 297 457
- US-B1- 6 387 077

## Description

### FIELD OF THE INVENTION

The present invention relates generally to medical devices, and more particularly to delivery devices for administration of pharmaceutical suspensions.

### BACKGROUND OF THE INVENTION

Pharmaceutical suspensions are widely used for different administration routes and may be broadly classified as injectable suspensions, oral suspensions, and topical suspensions.

In an ideal pharmaceutical suspension, the insoluble drug particles are uniformly dispersed in three dimensions throughout the carrier medium and remain so over time. Every two equal sized volumetric doses from the ideal pharmaceutical suspension will thus contain the same amount of drug and will give the same clinical effect to the recipient.

In practice, however, pharmaceutical suspensions are physically unstable. Absent agitation, the dispersed drug particles will settle out under influence of the gravitational force, and a sediment layer will form at the bottom of the container. This causes local changes of the drug concentration and accordingly, if unmitigated, non-uniform dosing. Particularly, a risk of significant under-dosing is introduced.

US 8,882,736 (Norton Healthcare Limited) discloses a compressible container for storing and dispensing a pharmaceutical suspension, which allows for easy re-suspension of particles that have settled out of the liquid during storage. By squeezing the container between two or more fingers the user is able to force the liquid through an orifice and into a spheroidal container portion which allows for the formation of a vortex, sufficient to re-suspend the sedimented particles.

Following a proper re-suspension of the drug particles, any metered dose should contain the intended amount of drug, and the risk of not receiving the correct dose is thereby eliminated. However, with a container of the type described in US 8,882,736 the user must remember to manipulate the liquid as prescribed prior to dose administration. Failure to do so will result in uncertainty regarding the actually dispensed amount of drug. Furthermore, elderly and others with reduced motor skills and finger strength may find the container difficult to handle, and these people are consequently at greater risk of not obtaining the right treatment.

US 2015/0297457 discloses a mixing syringe for delivery of a medical composition containing a contrast agent. The mixing syringe comprises an outer plunger and an inner plunger disposed at least partially within the outer plunger. The outer plunger comprises a plurality of annular ribs and one or more scraping elements to ensure sufficient mixing of the composition, including the re-suspension of any settled contrast agent.

Further examples of delivery systems that provide for re-suspension can be found in e.g. US 2015/0045773 and US 2014/0005610. US 2011/0270220 discloses a linearly actuated dispenser having a foil reservoir.

### SUMMARY OF THE INVENTION

It is an object of the invention to eliminate or reduce at least one drawback of the prior art, or to provide a useful alternative to prior art solutions. The invention is defined by the appended claims.

In particular, it is an object of the invention to provide a solution for administering a pharmaceutical suspension by which the risk of a user not receiving the intended dose of drug is minimised or eliminated.

It is a further object of the invention to provide a device or system for administering a pharmaceutical suspension which is safe and easy to handle, and which furthermore is reliable in relation to expressing the intended dose of drug.

In the disclosure of the present invention, aspects and embodiments will be described which will address one or more of the above objects and/or which will address objects apparent from the following text.

In a solution embodying the principles of the invention a dose delivery device comprises a variable volume reservoir holding a pharmaceutical suspension and comprising an outlet, a dose expelling mechanism adapted for activation to expel a volume of the pharmaceutical suspension through the outlet, and a dose preparation system operable prior to activation of the dose expelling mechanism to enable administration of the volume of the pharmaceutical suspension to a subject, and operation of the dose preparation system causes agitation of the pharmaceutical suspension.

Thereby, it is impossible to perform a dose administration with the dose delivery device without the pharmaceutical suspension becoming agitated first, and re-suspension is thus automatically ensured before the volume of the pharmaceutical suspension enters the body of the subject.

In one aspect, the invention accordingly provides a dose delivery device comprising a variable volume reservoir holding a pharmaceutical suspension and comprising an outlet, a dose expelling mechanism adapted for activation to expel a volume of the pharmaceutical suspension through the outlet, and a dose preparation system comprising a preparation member operable prior to activation of the dose expelling mechanism to enable administration of the volume of the pharmaceutical suspension to a subject. The dose preparation system further comprises an agitation member capable of agitating relative motion with respect to the variable volume reservoir, which agitating relative motion causes a re-suspending agitation of the pharmaceutical suspension. The agitation member is operatively coupled with the preparation member and configured to undergo said agitating relative motion in response to the operation of the preparation member.

Thereby, when the user operates the preparation member in preparation for a dose administration event, the pharmaceutical suspension becomes automatically agitated and thus readied for proper and reliable dosing. Since the operation of the preparation member is necessary to enable the administration of the volume of the pharmaceutical suspension to the subject, the dose delivery device provides a guarantee that the drug particles have been re-suspended once the dose expelling mechanism is activated, and the user does therefore not need to remember to perform a specific manual re-suspension action. Furthermore, as will be clear from the below, the operation of the preparation member can be ergonomically very simple and effortless to allow use also by people with reduced strength and dexterity.

The agitation member may be movable relative to the variable volume reservoir between a first position and a second position and may be configured to move between the first position and the second position, e.g. from the first position to the second position, in response to the operation of the preparation member. The first position may be a first predetermined position. Similarly, the second position may be a second predetermined position.

In exemplary embodiments of the invention the dose expelling mechanism is operatively coupled with the preparation member and configured to activate automatically in response to the operation of the preparation member, when or after the agitation member reaches the second position. For example, the preparation member may comprise a shield member carrying a magnet and being proximally displaceable relative to the variable volume reservoir from an outlet covering position to an outlet exposing position, the agitation member may comprise a magnetic element in the variable volume reservoir, the magnetic element being movable from a distal position to a proximal position in the variable volume reservoir in response to the shield member moving from the outlet covering position to the outlet exposing position, and the dose expelling mechanism may be spring powered and configured for release in response to the shield member reaching the outlet exposing position.

The agitation member may be movable relative to the variable volume reservoir along, at an angle to, such as e.g. perpendicularly to, and/or about a reference axis, i.e. the movement may be translational, rotational, or a combination of translational and rotational, e.g. helical, with respect to the reference axis. Alternatively, the agitation member may be movable relative to the variable volume reservoir along a first reference axis and about a second reference axis. The first reference axis and the second reference axis may be perpendicular. In any case, the first position and the second position may be respective axial, lateral and/or angular positions relative to the variable volume reservoir.

For example, the agitation member may extend along the reference axis and be configured to undergo the agitating relative motion by translating along and/or rotating about said reference axis relative to the variable volume reservoir between the first position and the second position in response to the operation of the preparation member.

The configuration of the agitation member may be selected by the manufacturer to produce a desired turbulence within the pharmaceutical suspension during the agitating relative motion with respect to the variable volume reservoir.

The preparation member may be operable prior to activation of the dose expelling mechanism to enable expelling of the volume of the pharmaceutical suspension through the outlet. In other words, the dose expelling mechanism may be prevented from expelling the volume of the pharmaceutical suspension through the outlet prior to the operation of the preparation member. In such case the dose delivery device may further comprise a releasable lock switchable from an initial state in which activation of the dose expelling mechanism is prevented to a released state in which activation of the dose expelling mechanism is enabled, where the releasable lock is operatively coupled with the preparation member and configured to switch from the initial state to the released state in response to the operation of the preparation member. Hence, the operation of the preparation member may comprise removal of a physical obstruction to movement of one or more parts of the dose expelling mechanism.

Such an arrangement will prevent the user from activating the dose expelling mechanism without previously operating the preparation member, and the risk of the user accidentally activating the dose expelling mechanism, e.g. as a mere consequence of the dose delivery device being carried about loosely in a purse or bag, and resultantly wasting drug to the surroundings is thereby eliminated.

The releasable lock may form part of the agitation member, whereby the number of different components in the dose delivery device is reduced.

The dose delivery device may further comprise a housing accommodating at least a portion of the dose expelling mechanism and defining a reference axis.

The variable volume reservoir may be any type of variable volume container suitable for holding the pharmaceutical suspension, such as e.g. a syringe having a staked needle, a cartridge type container comprising a generally cylindrical body which is sealed proximally by a slidable rubber stopper and has a necked down distal outlet portion which may be sealed by a pierceable septum, or a pouch type container comprising a deformable body with an integrated outlet portion.

In exemplary embodiments of the invention the variable volume reservoir is a deformable reservoir, such as a flexible foil reservoir, and the agitation member comprises a deformation element adapted to deform the flexible foil reservoir to thereby provoke the re-suspending agitation of the pharmaceutical suspension. The re-suspension can thereby be accomplished without the presence of a foreign object in the pharmaceutical suspension. The deformation element may be adapted to sweep and squeeze an outer surface of the flexible foil reservoir to provoke the re-suspending agitation of the pharmaceutical suspension, this providing for a mechanically simple and ergonomic construction. Alternatively, the deformation element may e.g. be adapted to squeeze different areas of the outer surface in a non-sweeping, predetermined or random sequence, or to rub the outer surface in a rotary motion. In any case, the impact on the outer surface will cause a compression of the flexible foil reservoir which in turn will cause a disturbance in the pharmaceutical suspension.

As used herein, the term "flexible foil reservoir", or simply "foil reservoir", designates a container which is capable of being deformed by the deformation element to obtain the re-suspending agitation, i.e. a container having one or more flexible surface portions. The "foil reservoir" may thus be fully flexible in the sense that every portion thereof is deformable, e.g. like a pouch, or it may be partially flexible in the sense that only some portions thereof are deformable, e.g. like a foil sheet welded, or otherwise sealingly attached, to a rigid base member. It may comprise an integrated outlet element, such as an injection needle, or it may be adapted to receive a separate outlet element.

The preparation member may comprise a cap removably attached to the housing to cover the outlet, the deformation element may be attached to, or form part of, the cap, and the cap may be adapted to be removed by relative axial motion with respect to the housing and the flexible foil reservoir, the deformation element thereby sweeping and squeezing the outer surface of the flexible foil reservoir. This provides for a simple and easy-to-use dose preparation system with a minimum number of components.

The agitation member may further comprise a second deformation element and a third deformation element arranged axially spaced apart from one another and from the deformation element, and at least two of the deformation elements intersect the reference axis at different angles. Deformation elements intersecting the reference axis at different angles will undergo different relative movements with respect to the outer surface of the flexible foil reservoir, and this will promote the turbulence created in the pharmaceutical suspension.

Alternatively, the preparation member may comprise a pull tab removably attached to the housing, the deformation element may be attached to, or form part of, the pull tab, and the pull tab may be adapted to be removed by relative transversal motion with respect to the housing and the flexible foil reservoir, the deformation element thereby sweeping and squeezing the outer surface of the flexible foil reservoir.

The agitation member may further comprise a second deformation element arranged transversally spaced apart from the deformation element, and the two deformation elements may intersect the reference axis at different angles, promoting the turbulence created in the pharmaceutical suspension.

The dose delivery device may further comprise a cap removably attached to the housing to cover the outlet, and the agitation member and the cap may comprise mutually interacting contact members configured to prevent removal of the cap when the pull tab is attached to the housing. Removal of the pull tab, causing automatic re-suspension, is thus required to expose the outlet and thereby enable administration of a dose to the user.

The dose expelling mechanism may comprise an actuator and a compression member adapted to collapse the flexible foil reservoir in response to an axial displacement of the actuator from a first axial position to a second axial position, and the agitation member may be configured to block movement of the actuator from the first axial position towards the second axial position when the pull tab is attached to the housing. This constitutes an example of the above-mentioned releasable lock, in this case forming part of the agitation member, where the removal of the pull tab switches the releasable lock from the initial state to the released state, thereby enabling activation of the actuator.

In other exemplary embodiments of the invention, in which the variable volume reservoir may be a deformable reservoir or a non-flexible reservoir, the agitation member is submerged in the pharmaceutical suspension and configured to travel within the variable volume reservoir to thereby provoke the re-suspending agitation of the pharmaceutical suspension.

In some such embodiments, the variable volume reservoir is a non-flexible reservoir, the agitation member is configured to promote turbulence in the non-flexible reservoir, and the preparation member is integrally or mechanically connected to the agitation member.

For example, the non-flexible reservoir may comprise an elastomeric piston having a central bore, and the dose expelling mechanism may comprise a piston rod structure for actuating the piston, where the piston rod structure comprises a) a shaft with a front shaft portion configured to extend, in a tight connection, through the central bore, and b) a drive tube abutting a proximal surface of the piston, and where the shaft is adapted to undergo initial proximal motion relative to the piston and the drive tube from a pre-use position to a dose ready position in which the drive tube engages the shaft, and subsequent joint distal motion with the piston and the drive tube. In that case, the preparation member may constitute an enlarged proximal end portion of the shaft configured for user operation, and the agitation member may constitute an enlarged distal end portion of the shaft configured to promote a swirling motion of the pharmaceutical suspension during the initial proximal motion.

Hence, the dose preparation system may form part of the dose expelling mechanism, as the preparation member, the agitation member, and the shaft may either be one unitary component or two or three mechanically coupled components, minimising the number of parts needed for the automatic re-suspension of the pharmaceutical suspension.

The non-flexible reservoir may be a syringe with a syringe barrel and a staked needle, whereby the need for preparatory needle handling actions is eliminated.

In other such embodiments, the agitation member is or comprises a magnetic element, and the preparation member is or comprises a magnet capable of affecting the position of the magnetic element in the variable volume reservoir. This enables a development of solutions where a re-suspending agitation of the pharmaceutical suspension is performed automatically by one or more actions carried out by the user as part of a customary use of the device, i.e. where no dedicated additional operations of the device are introduced to obtain the re-suspension.

For example, the outlet may comprise an injection needle with a needle end portion configured for insertion into skin, the preparation member may comprise a needle shield carrying the magnet and being proximally displaceable relative to the variable volume reservoir from a first shield position in which the needle end portion is covered to a second shield position in which the needle end portion is exposed, and the agitation member being or comprising the magnetic element may be movable from a distal position to a proximal position in the variable volume reservoir in response to the needle shield moving from the first shield position to the second shield position.

The proximal displacement of the needle shield which is necessary to expose the needle end portion and allow insertion thereof into the skin of the user thus ensures the re-suspending agitation of the pharmaceutical suspension by causing the agitation member inside the variable volume reservoir to follow the proximal motion of the carried magnet and thereby create turbulence in the pharmaceutical suspension.

In an auto-injector version of the dose delivery device the dose expelling mechanism may be spring powered and configured for release and automatic dose expelling in response to the needle shield reaching the second shield position. The very same movement that causes re-suspension of the pharmaceutical suspension thus also causes an automatic expelling of the pharmaceutical suspension immediately thereafter. This provides an easy-to-handle dose delivery device from which a dose can be administered by simply placing the needle shield at a desired location on the skin surface and pressing the variable volume reservoir towards the skin.

Alternatively, the preparation member may comprise an outlet protecting cap which must be removed from the variable volume reservoir to enable a dose expelling into the skin. The cap may carry the magnet and be removable by distal motion relative to the variable volume reservoir, and the agitation member being or comprising the magnetic element may be movable from a proximal position to a distal position in the variable volume reservoir in response to the cap being removed.

The distal displacement of the cap which is necessary to expose the outlet thus ensures the re-suspending agitation of the pharmaceutical suspension by causing the agitation member inside the variable volume reservoir to follow the distal motion of the carried magnet and thereby create turbulence in the pharmaceutical suspension. Following the cap removal, the dose delivery device may be ready for dose administration.

The agitation member may be shaped to optimise the conditions for generating turbulence in the pharmaceutical suspension. In particular, if the variable volume reservoir is a non-flexible reservoir, the agitation member may have an external dimension which substantially corresponds to an internal dimension of the non-flexible reservoir. For example, if the non-flexible reservoir comprises a circular-cylindrical reservoir body having an inner reservoir diameter, the agitation member may comprise an annular agitation member body having an outer agitation member diameter which is marginally smaller than the inner reservoir diameter. In that case, an outer surface portion of the agitation member body may be provided with one or more canals to allow passage of the pharmaceutical suspension along the agitation member body. One or more of the canals may extend axially or may be inclined with respect to the longitudinal axis defined by the reservoir body, the latter to induce a swirling motion of the liquid in the wake of the agitation member.

The agitation member body may further define a central bore, having an inner agitation member diameter, allowing for liquid passage therethrough, i.a. to reduce drag.

The agitation member body may be formed of a magnetic material. Alternatively, the agitation member body may comprise a magnetic core covered by a non-magnetic shell, e.g. of plastic.

In a variation of the above, the dose preparation system may comprise a first preparation member in the form of an outlet protecting cap carrying a first magnet capable of affecting the position of the magnetic element in the variable volume reservoir and a second preparation member in the form of a needle shield carrying a second magnet capable of affecting the position of the magnetic element in the variable volume reservoir. With an appropriate ratio of the individual magnet strengths, a first re-suspending agitation of the pharmaceutical suspension may be accomplished during removal of the outlet protecting cap, and a second re-suspending agitation of the pharmaceutical suspension may be accomplished subsequently during the proximal displacement of the needle shield.

As used herein, the term "pharmaceutical suspension" refers to any dosage form containing therapeutically active solid particles dispersed in a liquid medium, where the solid particles are sufficiently large for sedimentation. The term applies to such dosage form even in a state where the particles have settled out. Also, when a first denominated feature and a second denominated feature are said to be "operatively coupled", this means that the two are connected in a way to perform a designated function, and implies that a change of state, position and/or orientation of the one feature affects the state, position and/or orientation of the other. The term covers features that are integrally connected, in abutment, assembled, or configured for interaction at a distance, i.e. the features may be different but integral portions of one unitary piece, or they may be separate parts that are mechanically connected or otherwise influencing one another, e.g. contactlessly such as e.g. by magnetism.

For the avoidance of any doubt, in the present context the term "injection device" designates an apparatus suitable for injecting fluid media into the body of a subject, e.g. with the aid of an attachable needle device, and the term "drug" designates a medium which is used in the treatment, prevention or diagnosis of a condition, i.e. including a medium having a therapeutic or metabolic effect in the body. Further, the terms "distal" and "proximal" denote positions at, or directions along, a drug delivery device, a drug reservoir, or a needle unit, where "distal" refers to the drug outlet end and "proximal" refers to the end opposite the drug outlet end.

In the present specification, reference to a certain aspect or a certain embodiment (e.g. "an aspect", "a first aspect", "one embodiment", "an exemplary embodiment", or the like) signifies that a particular feature, structure, or characteristic described in connection with the respective aspect or embodiment is included in, or inherent of, at least that one aspect or embodiment of the invention, but not necessarily in/of all aspects or embodiments of the invention. It is emphasized, however, that any combination of the various features, structures and/or characteristics described in relation to the invention is encompassed by the invention unless expressly stated herein or clearly contradicted by context.

The use of any and all examples, or exemplary language (e.g., such as, etc.), in the text is intended to merely illuminate the invention and does not pose a limitation on the scope of the same, unless otherwise claimed. Further, no language or wording in the specification should be construed as indicating any non-claimed element as essential to the practice of the invention.

### BRIEF DESCRIPTION OF THE DRAWINGS

In the following the invention will be further described with references to the drawings, wherein
Fig. 1 is an exploded view of a dose delivery device according to a first exemplary embodiment of the invention,
Fig. 2 is a perspective view of the dose delivery device in a pre-use state,
Figs. 3 and 4 are respective perspective and longitudinal section views of the dose delivery device during initial cap dismounting,
Fig. 5 is a schematic representation of the re-suspension principle employed in the dose delivery device,
Figs. 6-11 are different views of the dose delivery device in various operational states during dose preparation and dose administration,
Fig. 12 is an exploded view of a dose delivery device according to a second exemplary embodiment of the invention,
Fig. 13 is a perspective view of the dose delivery device in a pre-use state,
Figs. 14-19 are different views of the dose delivery device of Fig. 13 in various operational states during dose preparation and dose administration,
Fig. 20 is a longitudinal section view of a dose delivery device according to a third exemplary embodiment of the invention,
Figs. 21 and 22 are longitudinal section views of the dose delivery device of Fig. 20 following, respectively, dose preparation and dose administration,
Fig. 23 is an exploded view of a dose delivery device according to a fourth exemplary embodiment of the invention,
Figs. 24-27 are different views detailing various components of the dose delivery device of Fig. 23,
Figs. 28-37 are different perspective views of the dose delivery device of Fig. 23 in various operational states during dose preparation and dose administration, where a portion of the device has been cut away for clarity,
Figs. 38 and 39 are respective exploded views of sub-assemblies of a dose delivery device according to a fifth exemplary embodiment of the invention,
Fig. 40 is a longitudinal section view of the sub-assembly of Fig. 38,
Figs. 41-46 are various views of individual components of the sub-assemblies,
Fig. 47 is a perspective view of the sub-assemblies before final assembly of the dose delivery device, with various portions being cut away or made transparent for clarity,
Figs. 48-60 are different views of the dose delivery device in various operational states during dose preparation and dose administration, and
Figs. 61-64 are close up views of the dose release mechanism.

In the figures like structures are mainly identified by like reference numerals.

### DESCRIPTION OF EXEMPLARY EMBODIMENTS

When/If relative expressions, such as "upper" and "lower", "left" and "right", "horizontal" and "vertical", "clockwise" and "counter-clockwise", etc., are used in the following, these refer to the appended figures and not necessarily to an actual situation of use. The shown figures are schematic representations for which reason the configuration of the different structures as well as their relative dimensions are intended to serve illustrative purposes only.

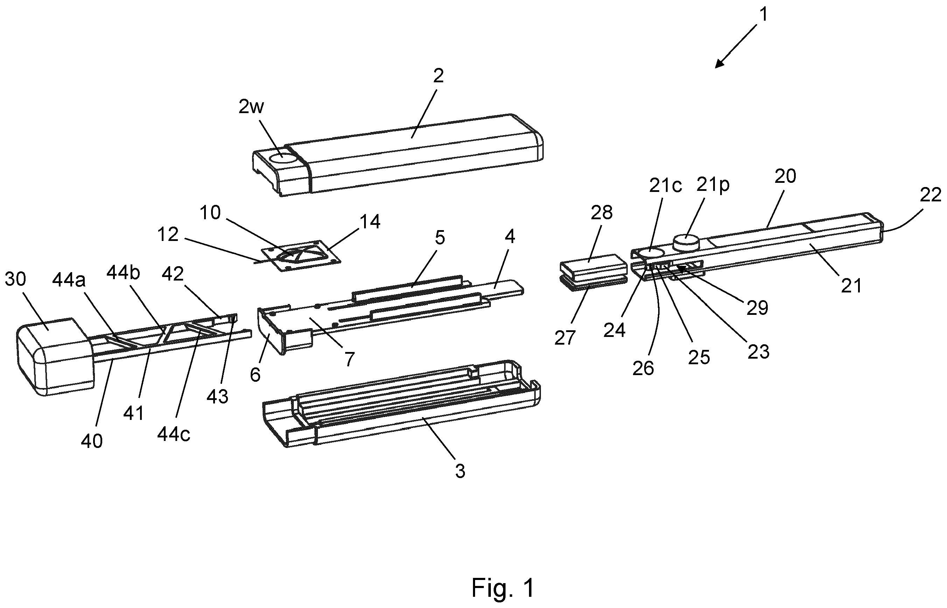

Fig. 1 is an exploded view of a dose delivery device 1 according to a first exemplary embodiment of the invention. The dose delivery device 1 comprises a rigid base member 4 arranged in a housing 2, 3 defined by a top shell 2 and a bottom shell 3. A flexible foil reservoir 10 with an integrated injection needle 12 and a carrier board 14 is fixed to a front portion 7 of the base member 4, just proximally of a transversal end plate 6. The foil reservoir 10 holds a volume of a pharmaceutical suspension. A pair of axially extending rails 5 are arranged along opposite sides of the base member 4.

The dose delivery device 1 further comprises a cap 30 to which an axially extending rib structure 40 is attached. The rib structure 40 comprises two parallel side members 41 and three ribs 44a, 44b, 44c. A distal rib 44a connects the side members 41 at a first inclined angle, a middle rib 44b connects the side members 41 at a second inclined angle, and a proximal rib 44c connects the side members 41 at a third inclined angle, identical to the first inclined angle. Each of the side members 41 has a thinned proximal section 42 carrying a v-shaped hook 43.

The dose delivery device 1 also comprises a dose expelling member 20 having an elongated body 21 with a proximal push button 22 and a distal recess 29 adapted to accommodate a two-part rubber squeezer 27, 28. A colour marking 21c is positioned on a top surface of the elongated body 21 just distally of a protruding guide member 21p, and two lateral protrusions 23, 24 are arranged in axial extension of one another on either side of the elongated body 21 (only one pair is visible). One of the lateral protrusions 23 has a skewed leading face 25 and the other lateral protrusion 24 has a skewed trailing face 26, leaving a v-shaped indentation between the two lateral protrusions 23, 24. The protruding guide member 21c is adapted to provide for linear advancement of the dose expelling member 20 into the housing 2, 3, and the colour marking 21c is arranged to become visible to the user through a window 2w in the top shell 2 when the dose expelling member 20 is fully advanced into the housing 2, 3 to thereby visually signal that a dose administration action has been properly carried out.

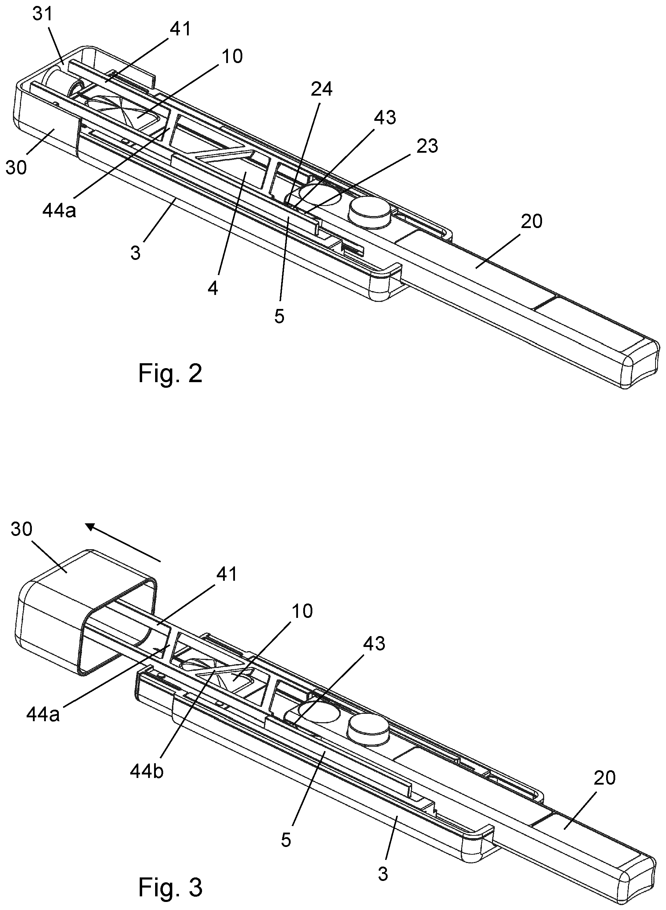

Fig. 2 is a perspective view of the dose delivery device 1 in a pre-use state, with the top shell 2 removed for the sake of clarity. In this state the cap 30 is fully mounted on the housing 2, 3 and thus covers and protects the injection needle 12. As can be seen, the side members 41 are fixedly attached to an internal end wall 31 of the cap 30, e.g. by gluing or welding, and the distal rib 44a is positioned proximally of the foil reservoir 10. It is also seen that the rib structure 40 and the dose expelling member 20 are connected in that the hooks 43 (only one is visible) are trapped between the respective lateral protrusions 23, 24 and the rails 5.

The following figures show the dose delivery device 1 in various operational states. To prepare for an administration of the volume of the pharmaceutical suspension contained in the foil reservoir 10 the cap 30 must initially be pulled off the housing 2, 3, in the direction of the arrow as shown in Fig. 3. This leads to the ribs 44a, 44b, 44c successively sweeping the foil reservoir 10, thereby inducing a re-suspending agitating motion of the pharmaceutical suspension, as well as to the dose expelling member 20 being pulled into the housing 2, 3 due to the engagement between the hooks 43 and the lateral protrusions 23, 24. During the distal displacement of the cap 30 the skewed trailing face 26 applies a reaction force to the hook 43, which reaction force has a small non-axial component that points radially outwardly. However, so long as the hook 43 moves along the rail 5 the hook 43 is prevented from disengaging from the indentation between the two lateral protrusions 23, 24. In Fig. 3 the distal rib 44a has swept the foil reservoir 10 completely and the middle rib 44b is about halfway through.

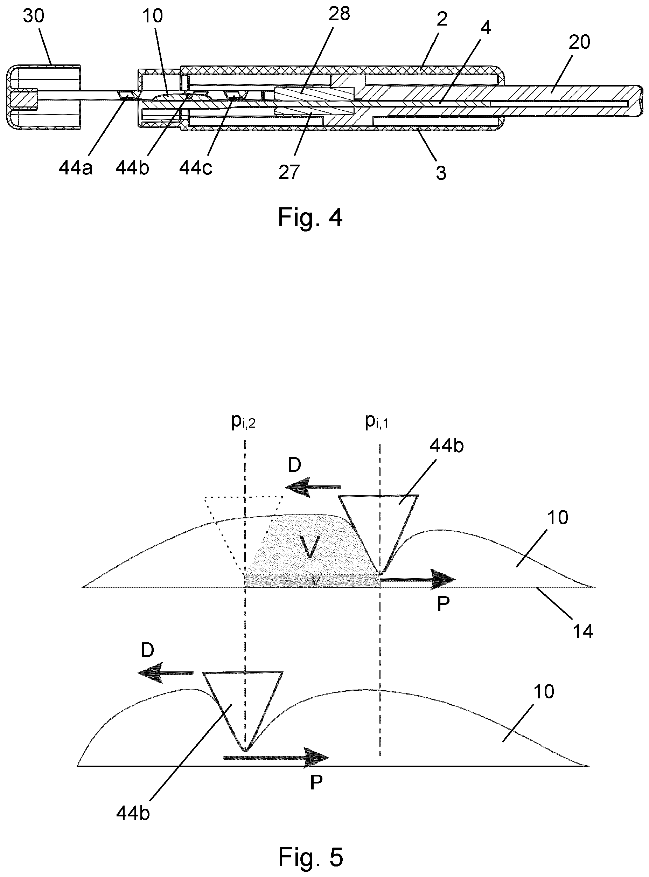

Fig. 4 is a longitudinal section view of the dose delivery device 1 in the state shown in Fig. 3, showing the two parts of the rubber squeezer 27, 28 arranged on each side of the base member 4. As the dose expelling member 20 is pulled distally by the movement of the cap 30 the two parts of the rubber squeezer 27, 28 slide along the base member 4 and approach the foil reservoir 10.

Fig. 5 is a schematic illustration of the re-suspension principle employed by the dose delivery device 1. Using a flexible reservoir enables re-suspension of drug particles in the liquid by applying pressure to the foil and changing the location of the applied pressure to mechanically provoke a turbulent motion of the liquid. A turbulent motion is preferable for two reasons. Firstly, it ensures a much better mixing and thus more uniform concentration, and secondly it involves higher velocities and thinner boundary layers which causes the liquid to stir up particles closer to the surface than laminar flow would.

A high velocity turbulent flow is induced in the foil reservoir 10 by applying a pressure to an area and, while continuously applying this pressure, moving the area relative to the foil. As pressure is applied to an area, liquid is displaced from that area and moves to somewhere else within the foil reservoir 10. Fig. 5 shows the principle of what happens as the middle rib 44b moves in the axial direction over the foil reservoir 10, although for simplicity the middle rib 44b is here sketched as having an axis of extension perpendicular to the axis of motion.

In any interacting position the middle rib 44b makes a dent in the liquid-filled foil reservoir 10. As the middle rib 44b is moved with a velocity v from a first intermediate position, p_{i,1}, to a second intermediate position, p_{i,2}, a volume V of the liquid, indicated by the light grey colouring in Fig. 5, will be displaced and forced to move underneath the rib apex by the pressure buildup in front of the middle rib 44b. This volume V can only pass through the small area V underneath the rib apex, indicated by the darker grey colouring in Fig. 5, which in the present example is approximately ¼ of the volume V. Consequently, the average velocity of the liquid passing underneath the rib apex will be about 4v.

Due to the boundary conditions the velocity profile of the liquid is parabolic with a velocity of 0 at the interface to the rigid carrier board 14 and a maximum velocity which is significantly higher than four times the velocity of the middle rib 44b. Thereby, even a moderate rib velocity will generate a high velocity turbulent flow of the liquid passing underneath the rib apex. This high velocity flow enters the liquid present behind the middle rib 44b and causes turbulence in this volume as well.

So, with three ribs 44a, 44b, 44c thus sweeping the foil reservoir 10 in succession as the cap 30 is removed from the housing 2, 3 the pharmaceutical suspension becomes violently agitated and is consequently automatically re-suspended.

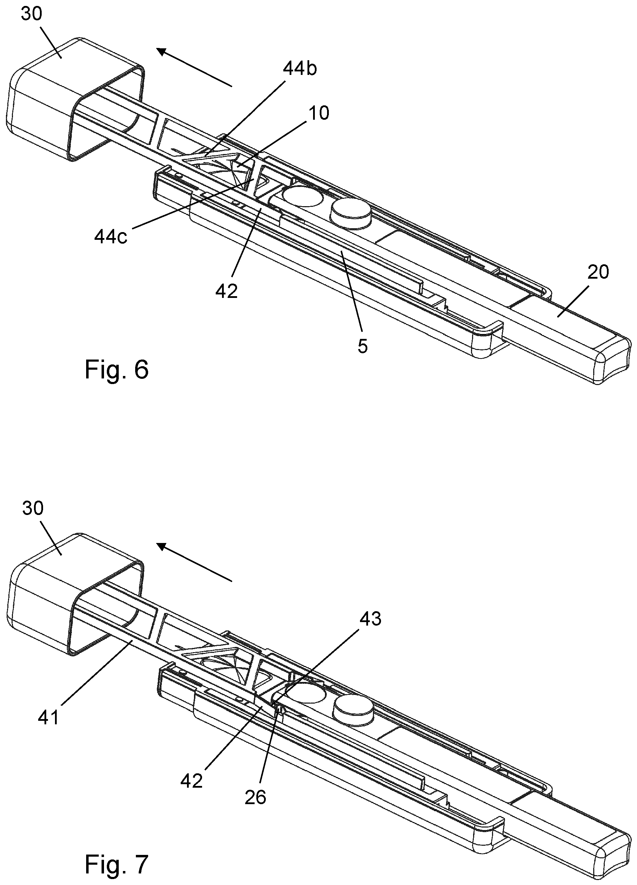

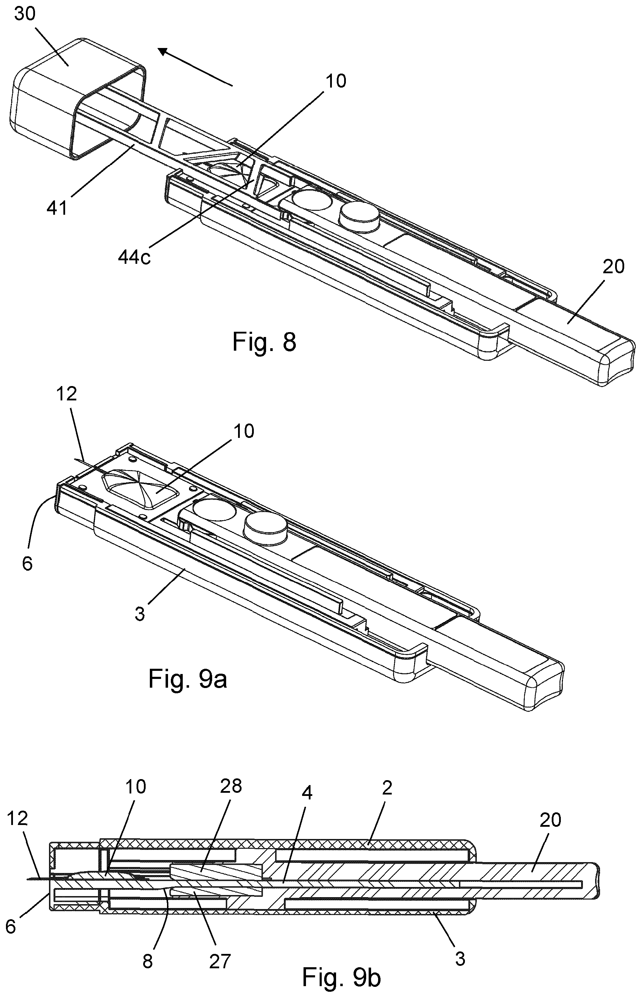

In Fig. 6 the cap 30 has been pulled distally to a point where the middle rib 44b has swept the foil reservoir 10 entirely and the proximal rib 44c has just initiated its interaction with the foil. Meanwhile, the thinned proximal sections 42 of the side members 41 have moved out of contact with the rails 5. At this point the rails 5 no longer prevent transversal motion of the thinned proximal sections 42, and a continued pulling of the cap 30 therefore leads to the thinned proximal sections 42 bending outwardly in response to the respective non-axial reaction force components from the skewed trailing face 26. This is illustrated in Fig. 7. Resultantly, the hooks 43 disengage from the respective indentations between the lateral protrusions 23, 24 and the cap 30 thus disengages from the dose expelling member 20, as seen in Fig. 8. During the last part of the dismounting movement of the cap 30 the dose expelling member 20 consequently remains stationary, as the proximal rib 44c sweeps the foil reservoir 10.

Figs. 9a and 9b show the remaining part of the dose delivery device 1 after removal of the cap 30. It can be seen from Fig. 9b that the rubber squeezer 27, 28 is positioned at a transition portion 8 where the thickness of the base member 4 gradually increases. This gradual increase in thickness provides an increased resistance to distal motion of the rubber squeezer 27, 28 along the base member 4 and thus contributes to an easy detachment of the cap 30 from the dose expelling member 20, as the force required to pull the dose expelling member 20 any further becomes greater than what is possible to transfer in the interface between the transversally freed hooks 43 and the indentations between the lateral protrusions 23, 24.

The transition portion 8 is, however, easy for the rubber squeezer 27, 28 to pass when being pushed by the elongated body 21. So, when the user subsequently inserts the injection needle 12 into the skin and depresses the push button 22 towards the housing 2, 3 to perform a dose administration the rubber squeezer 27, 28 easily overcomes the increased thickness of the base member 4 and continues towards the foil reservoir 10 without requiring much effort.

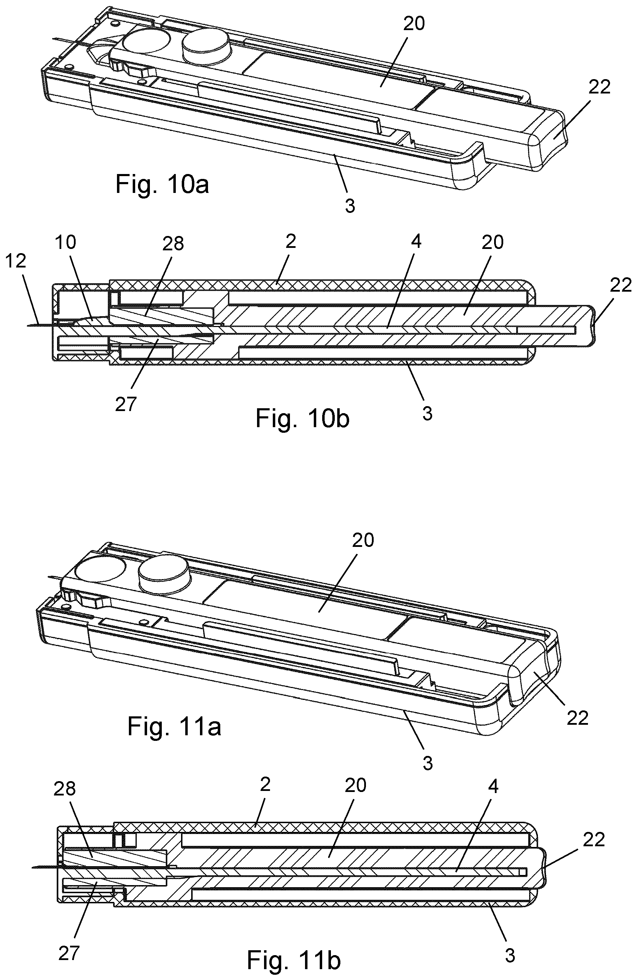

The increased thickness of the base member 4 at the front portion 7 leads to the rubber squeezer 27, 28 becoming slightly elastically deformed upon passage of the transition portion 8, which in turn leads to the rubber squeezer 27, 28 applying a greater compression force to the front portion 7 and, eventually, the foil reservoir 10, as the dose expelling member 20 is pressed further into the housing 2, 3. Hence, as the dose expelling member 20 advances distally into the housing 2, 3, illustrated by the different views in Figs. 10 and 11, the foil reservoir 10 is firmly compressed and collapses increasingly, whereby the pharmaceutical suspension is pressed out through the injection needle 12 and into the body of the user.

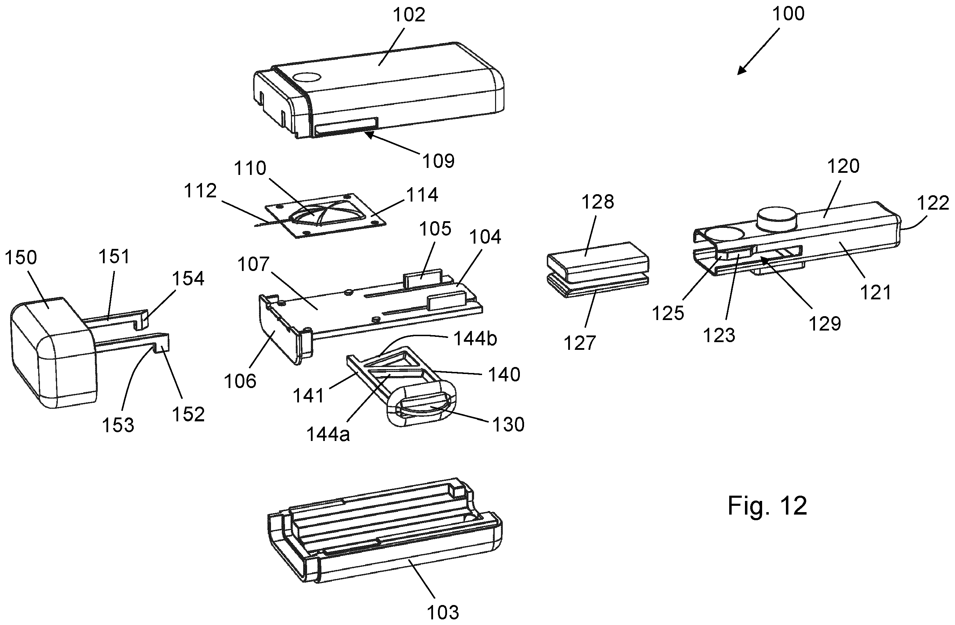

Fig. 12 is an exploded view of a dose delivery device 100 according to a second exemplary embodiment of the invention. The dose delivery device 100, which can be seen as a compact variant of the above described dose delivery device 1, comprises a rigid base member 104 arranged in a housing 102, 103 defined by a top shell 102 and a bottom shell 103. A flexible foil reservoir 110 with an integrated injection needle 112 and a carrier board 114 is fixed to a front portion 107 of the base member 104, just proximally of a transversal end plate 106. The foil reservoir 110 holds a volume of a pharmaceutical suspension. A pair of axially extending rails 105 are arranged along opposite sides of the base member 104.

The dose delivery device 100 further comprises a pull tab 130 to which a transversally extending rib structure 140 is attached. The rib structure 140 comprises two parallel side members 141, which are adapted to extend through an opening 109 in the top shell 102, and two ribs 144a, 144b. A front rib 144a connects the side members 141 at a first inclined angle, and a rear rib 144b connects the side members 141 at a second inclined angle, which is different from the first inclined angle.

The dose delivery device 100 also comprises a removable cap 150 and a dose expelling member 120 having an axially extending body 121 with a proximal push button 122 and a distal recess 129 adapted to accommodate a two-part rubber squeezer 127, 128. A lateral protrusion 123 is arranged on either side of the elongated body 121 (only one is visible). Each of the lateral protrusions 123 has a skewed leading face 125. The cap 150 comprises a pair of axially extending parallel arms 151 which each terminate in an enlarged end section 152 having a skewed proximal face 154 and a straight distal face 153.

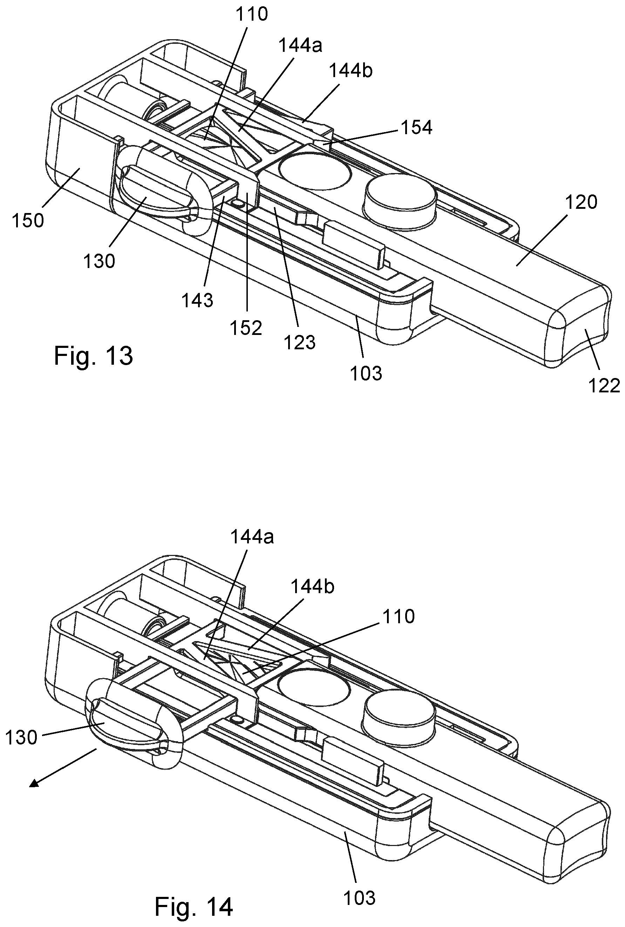

Fig. 13 is a perspective view of the dose delivery device 100 in a pre-use state, with the top shell 102 removed for the sake of clarity. In this state the cap 150 is fully mounted on the housing 102, 103 and thus covers and protects the injection needle 112. The arms 151 extend over the rib structure 140, and the straight distal face 153 of the enlarged end sections 152 abut a proximal side 143 of one of the side members 141 such that axial motion of the cap 150 relative to the housing 102, 103 is prevented. Furthermore, the skewed leading faces 125 of the lateral protrusions 123 abut the skewed proximal faces 154 of the enlarged end sections 152, and the pre-use state of the dose delivery device 100 is thus effectively a locked state in which the presence of the rib structure 140 prevents both a removal of the cap 150 and an activation of the dose expelling member 120.

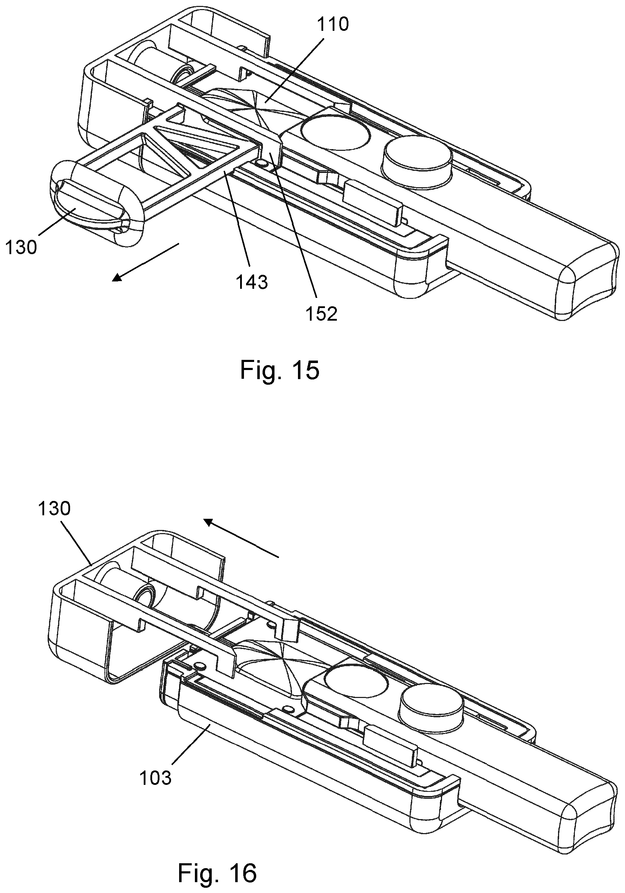

The following figures show the dose delivery device 100 in various operational states. To prepare for an administration of the volume of the pharmaceutical suspension contained in the foil reservoir 110 the pull tab 130 must initially be pulled out of the housing 2, 3, in the direction of the arrow, as shown in Fig. 14 and Fig. 15. This leads to the ribs 144a, 144b successively sweeping the foil reservoir 110, thereby inducing a re-suspending agitating motion of the pharmaceutical suspension in a similar fashion as described above in connection with the first exemplary embodiment of the invention.

Once the pull tab 130 is completely removed, and the proximal side 143 thus no longer obstructs movement of the enlarged end sections 152, the cap 130 can be detached from the housing 102, 103 by axial relative motion in the direction of the arrow shown in Fig. 16. This exposes the injection needle 112 and the dose delivery device 100 is thus ready for dose administration with a duly re-suspended drug.

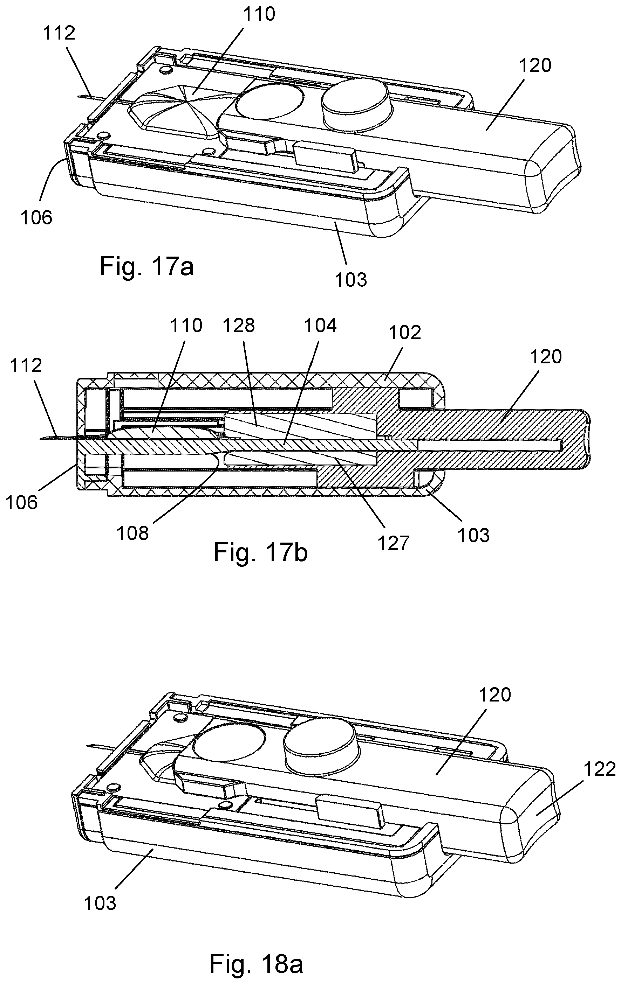

Figs. 17a and 17b depict the dose delivery device 100 in the ready state, and it can be seen from Fig. 17b that the rubber squeezer 127, 128 initially is positioned at a transition portion 108 where the thickness of the base member 104 gradually increases. Thereby, as was the case with the former exemplary embodiment of the invention, when the user inserts the injection needle 112 into the skin and depresses the push button 122 towards the housing 102, 103 to perform a dose administration the rubber squeezer 127, 128 becomes slightly elastically deformed upon passage of the transition portion 108 and therefore applies an increased compression force to the front portion 107 and the foil reservoir 110, as the dose expelling member 120 is pressed into the housing 102, 103.

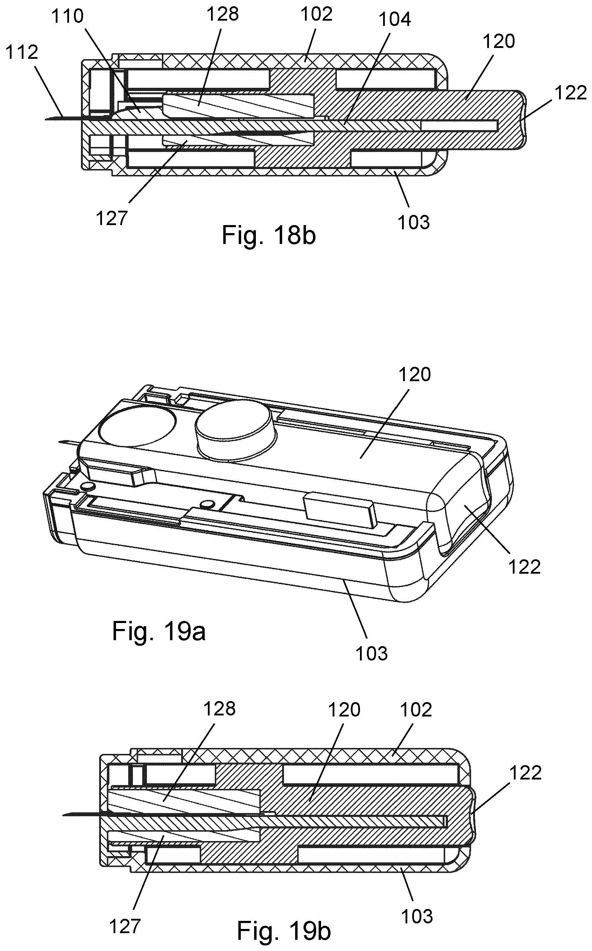

Hence, as the dose expelling member 120 advances distally into the housing 102, 103, illustrated by the different views in Figs. 18 and 19, the foil reservoir 110 is firmly compressed and collapses increasingly, whereby the pharmaceutical suspension is pressed out through the injection needle 112 and into the body of the user.

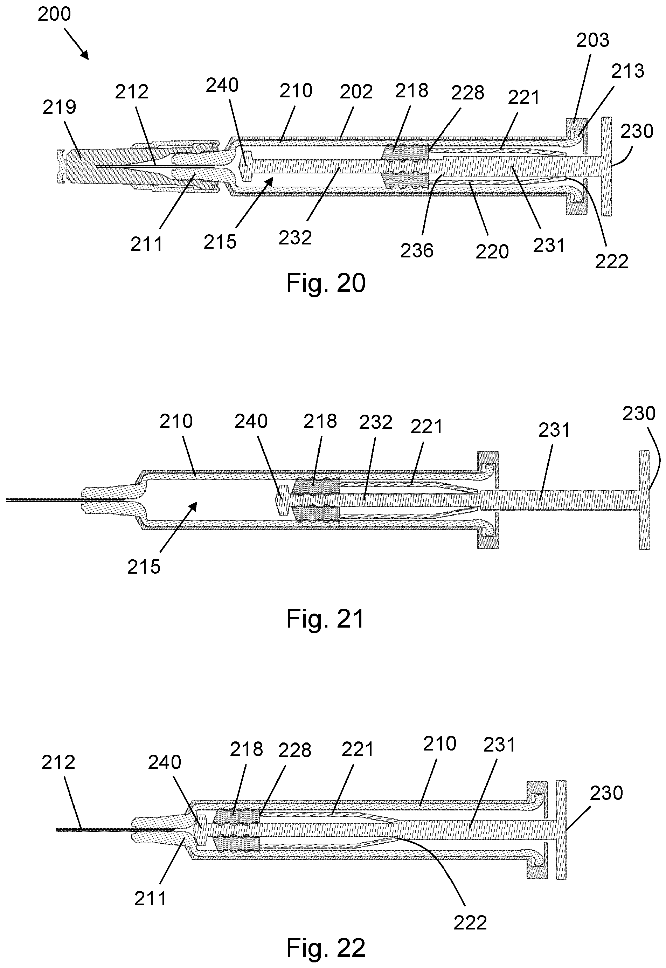

Fig. 20 is a longitudinal section view of a dose delivery device 200 according to a third exemplary embodiment of the invention. The dose delivery device 200 comprises a housing 202 which accommodates a syringe barrel 210. The syringe barrel 210 has a proximal collar 213 which is in locked engagement with a proximal housing end 203 and a distal outlet end portion 211 to which an injection needle 212 is fixedly attached. The injection needle 212 is in a pre-use state of the dose delivery device 200 covered by a removable protective cap 219.

A sealing rubber piston 218 separates an interior of the syringe barrel 210 in two, a wet chamber 215 which is prefilled with a pharmaceutical suspension and a dry chamber which accommodates a piston drive tube 220. The piston drive tube 220 has an axially rigid drive tube body 221 with a distal body end 228 in abutment with a proximal end face of the piston 218. A proximal end section of the drive tube body 221 tapers towards the centre of the syringe barrel 210 and terminates at a proximal body end 222.

The piston 218 has a central bore through which a centre shaft 232 extends in fluid tight manner. The centre shaft 232 has a radially enlarged shaft section 231 in the dry chamber and a distal mixing head 240 in the wet chamber 215. The enlarged shaft section 231 terminates proximally in a user operable pull-push knob 230 exteriorly of the housing 202 and distally in a transition 236. The radial dimension of the enlarged shaft section 231 is greater than the radial extent of the proximal body end 222 in a relaxed state of the drive tube body 221. This means that the proximal body end 222 is biased radially inwardly in the pre-use state of the dose delivery device 200.

The mixing head 240 has a radial dimension which is slightly smaller than the internal diameter of the syringe barrel 210. In the pre-use state shown in Fig. 20 the centre shaft 232 extends almost completely into the wet chamber 215, and the mixing head 240 is positioned close to the outlet end portion 211. Correspondingly, the pull-push knob 230 is positioned close to the proximal housing end 203. The centre shaft 232, including the enlarged shaft section 231, the mixing head 240, and the pull-push knob 230 is provided as one unitary component.

In operation, following a removal of the protective cap 219 from the outlet end portion 211, the user pulls back the centre shaft 232, by operation of the pull-push knob 230, until the mixing head 240 reaches the piston 218, as shown in Fig. 21. The proximal motion of the mixing head 240 relative to the syringe barrel 210 causes an agitating motion (not visible) of the pharmaceutical suspension around the mixing head 240 which is sufficient to re-suspend any sedimented drug particles. As the mixing head 240 reaches the piston 218 the transition 236 passes the proximal body end 222 which consequently snaps into a smaller diameter position, thereby axially locking the piston 218 and the piston drive tube 220 between the mixing head 240 and the transition 236.

To administer the re-suspended pharmaceutical suspension the user now pushes the pull-push knob 230 distally towards the proximal housing end 203, which causes the transition 236 to apply an axial drive force to the proximal body end 222, which drive force is transferred via the axially rigid drive tube body 221 to the piston 218. As the piston 218 advances through the syringe barrel 210 a volume of the pharmaceutical suspension is expelled through the injection needle 212. In Fig. 22 the pull-push knob 230 has been fully depressed and the piston 218 has reached its final, end-of-dose position in the syringe barrel 210.

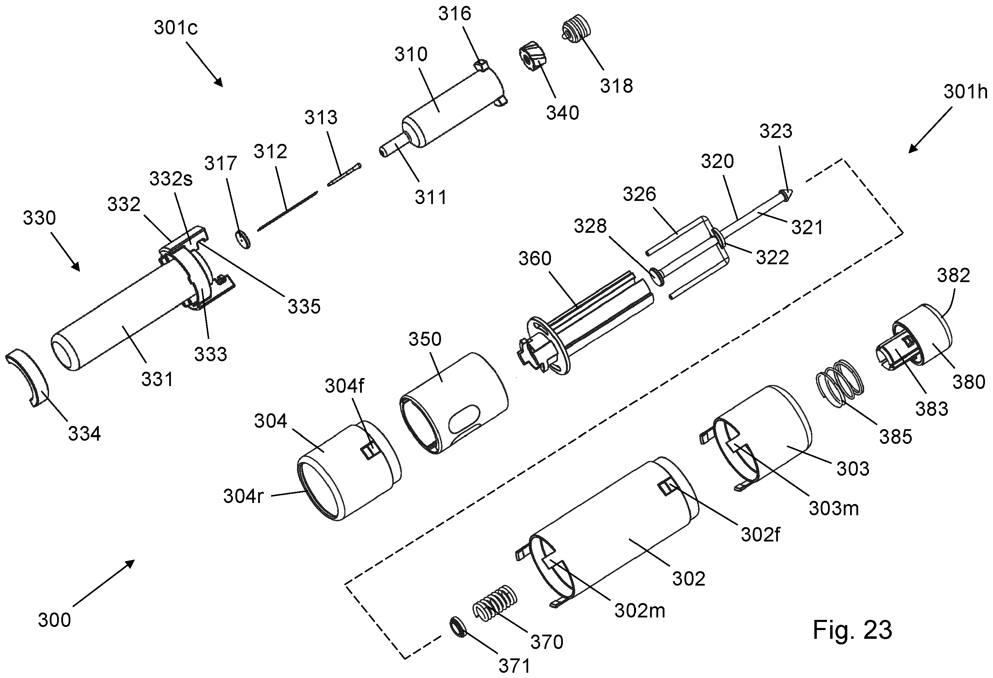

Fig. 23 is an exploded view of a dose delivery device 300 according to a fourth exemplary embodiment of the invention. The dose delivery device 300 is based on a cartridge type reservoir and comprises a cartridge assembly 301c and a housing assembly 301h. The cartridge assembly 301c comprises a cartridge 310 with a distal outlet end portion 311 and a pair of diametrically opposite proximal cartridge flanges 316. The cartridge 310 holds a volume of a pharmaceutical suspension and is sealed proximally by a slidable piston 318 and distally by a penetrable septum 313 adhered to the outlet end portion 311 in which also an injection needle 312 is arranged. A disc 317 is fixedly mounted on the injection needle 312 at a position which defines the possible depth of insertion of the injection needle 312 in the skin.

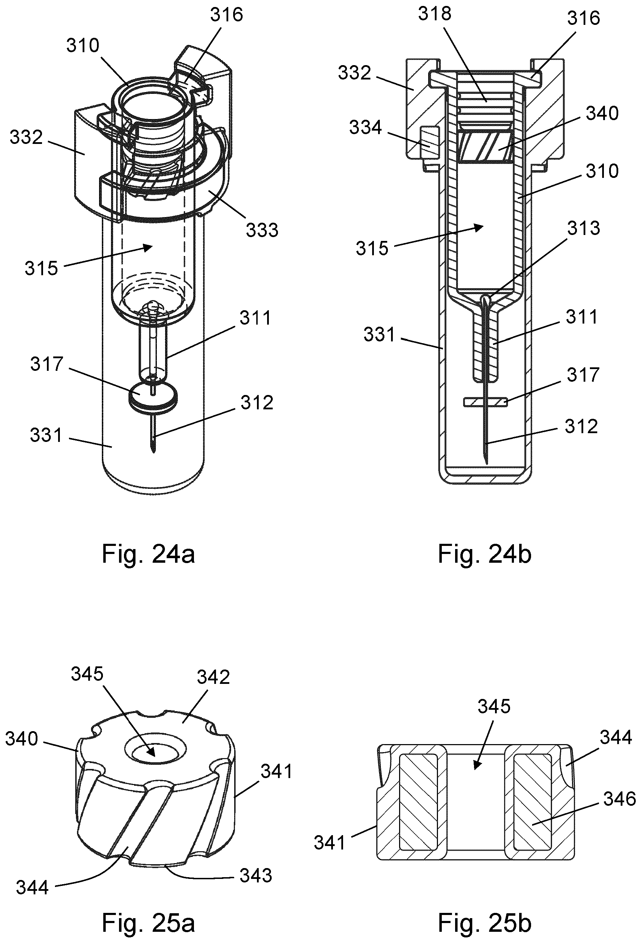

The cartridge assembly 301c further comprises a needle cap 330 which has a hollow needle cap body 331 adapted to accommodate the injection needle 312 and the cartridge 310. At its proximal end the hollow needle cap body 331 is provided with a pair of diametrically opposite needle cap flanges 332 which are connected by a semi-circular overhang 333 adapted to cover and hold a semi-circular magnet 334. The magnet 334 is adapted to attract a magnetic mixer element 340 arranged in an interior 315 (see Figs. 24a and 24b) of the cartridge 310. Each needle cap flange 332 has an abutment surface 332s and is provided with a notch 335 on an inner surface portion for rotational sliding reception of one of the cartridge flanges 316.

The housing assembly 301h comprises a three-part outer housing which consists of a central housing part 302, a proximal housing part 303, and a distal housing part 304. The outer housing is so divided to allow for positioning of internal components. Each housing part has means for fixed attachment to at least one of the other housing parts. Specifically, the proximal housing part 303 has a number of distally extending snap arms 303m which are each adapted to engage with one of a corresponding number of receiving indentations 302f in the central housing part 302, and the central housing part 302 similarly has a number of distally extending snap arms 302m which are each adapted to engage with one of a corresponding number of receiving indentations 304f in the distal housing part 304. The distal housing part 304 further has a radially inwardly protruding distal rim 304r.

The outer housing accommodates a lock ring 350, a stator 360, a piston rod 320, a drive spring 370 and a distal spring base 371. The piston rod 320 has a slender piston rod body 321, a distal piston rod foot 328, adapted to interface with the piston 318, a central plate 322 for supporting the distal spring base 371, and a proximal stud 323 for interaction with an interior portion of the proximal housing part 303. Just distally of the central plate 322 the piston rod 320 is provided with a hanger profile having axially extending arms 326. A push button 380 extends proximally from the proximal housing part 303. The push button 380 has a transversal end surface 382, adapted to interact with a finger, and an axially projecting stalk 383 and is biased in the proximal direction by a button spring 385.

Figs. 24a and 24b are respective perspective and longitudinal section views of the cartridge assembly 301c in a pre-use state, showing the cartridge 310 inside the hollow needle cap body 331. In the pre-use state, the cartridge flanges 316 are received in the notches 335, and the cartridge 310 is thereby axially fixed with respect to the needle cap 330. The position of the magnet 334 in the overhang 333 causes the magnetic mixer element 340 to maintain a proximal end position within the cartridge 310, next to the piston 318.

Figs. 25a and 25b are respective perspective and longitudinal section views of the magnetic mixer element 340, which has a ring-shaped plastic body 341 of an outer diameter essentially corresponding the inner diameter of the cartridge 310, with a central through-going bore 345 and an iron core 346. A plurality of canals 344 are formed on the exterior surface of the plastic body 341. Each canal 344 extends between a proximal mixer end surface 342 and a distal mixer end surface 343 and is inclined with respect to a longitudinal axis defined by the cartridge 310 and the injection needle 312.

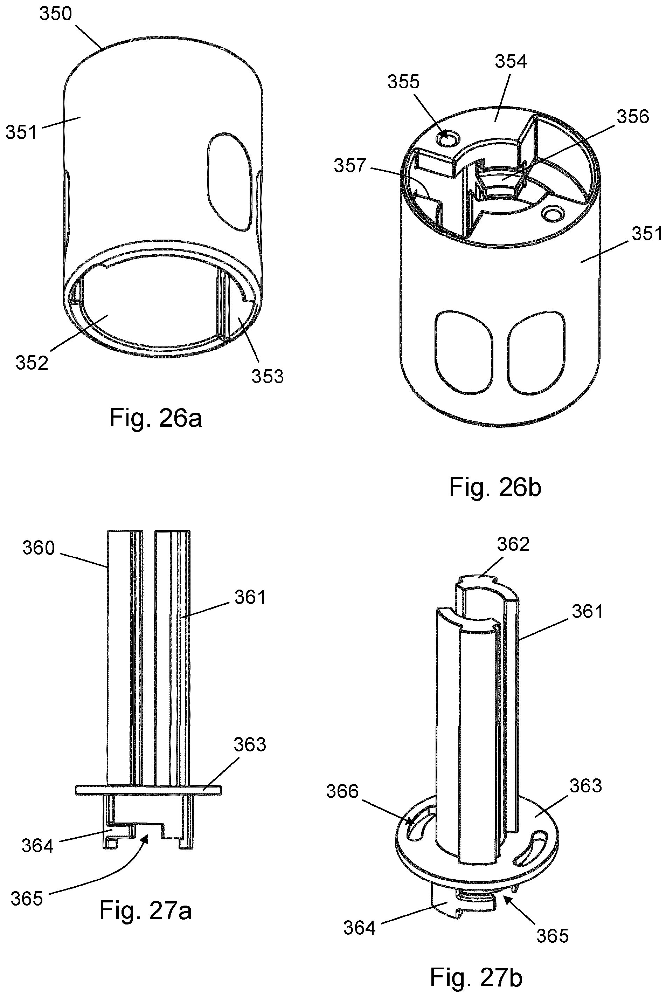

Figs. 26a and 26b are different perspective views of the lock ring 350. The lock ring 350 comprises a cylindrical lock ring body 351 with an inner surface 352 in which two longitudinal tracks 353 are provided for sliding reception of the respective needle cap flanges 332. In a proximal end portion the lock ring 350 has a pair of diametrically opposite interior shelves 356 (only one is visible) as well as a pair of diametrically opposite plateaus 354 provided with diametrically opposite bores 355 configured for reception of the respective arms 326 to rotationally interlock the hanger profile and the lock ring 350. The interior of the lock ring 350 is also provided with vertical reaction surfaces 357 (only one is visible) for interaction with the respective abutment surfaces 332s.

Figs. 27a and 27b are, respectively, a side view and a perspective view of the stator 360 which comprises a stator base 363 supporting two proximally extending curved pillars 361, each having a radially outwardly projecting key 362 along its entire length. The stator 360 further comprises a distal lock geometry 364 with two openings 365 (only one is visible) for reception and rotational fixation of the respective cartridge flanges 316. Two diametrically opposite curved slots 366 are provided in the stator base 363 which allow for passage of the respective arms 326 and accordingly, together with a circumferential spacing between the curved pillars 361, for predefined angular movement of the hanger profile relative to the stator 360.

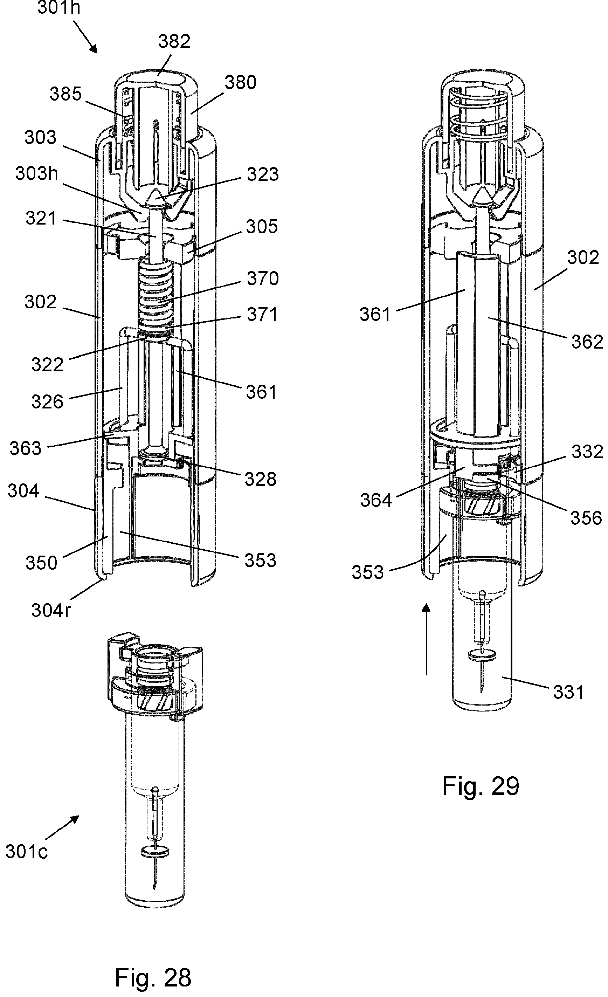

In the following an operation of the dose delivery device 300 will be described with reference to Figs. 28-37.

Fig. 28 is a perspective view of the dose delivery device 300 showing the cartridge assembly 301c ready for insertion into the housing assembly 301h. A portion of the housing assembly 301h has been removed to enable inspection of the interior components. It is seen that the lock ring 350 and the stator 360 are axially fixed relative to the outer housing between the distal rim 304r and a proximal spring base 305. The stator 360 is further rotationally fixed relative to the outer housing due to the keys 362 being rotationally locked in the proximal spring base 305. Furthermore, the proximal stud 323 is engaged with a pair of flexible fingers 303h which hold the piston rod 320 in position against the bias force from the pre-strained drive spring 370.

Fig. 29 shows the dose delivery device 300 after the initial step of linear insertion of the cartridge assembly 301c into the housing assembly 301h. During the insertion the needle cap flanges 332 slide in the respective longitudinal tracks 353 until they meet an axial stop in the lock ring 350 (not shown). When this happens the cartridge flanges 316 have entered the respective openings 365 in the stator 360. In this view the stator 360 is shown in full and some of the interior configuration of the lock ring 350 is made visible, in particular to enable identification of one of the shelves 356.

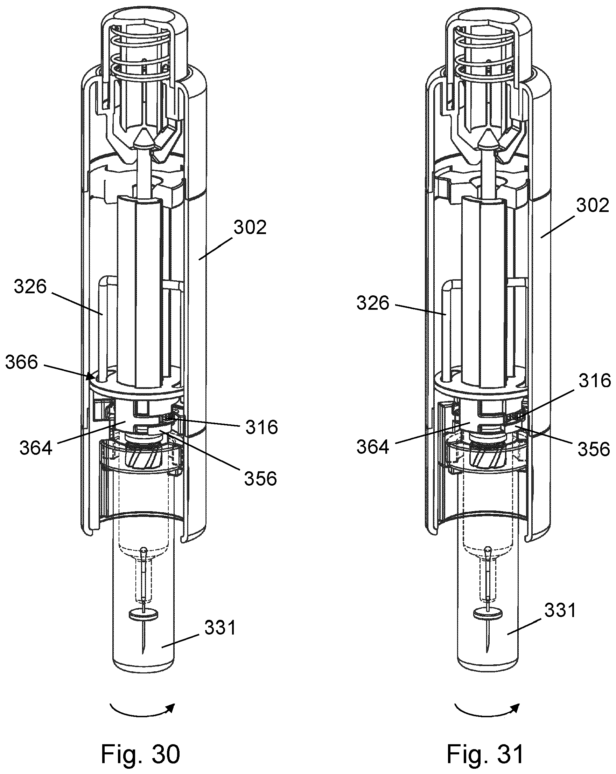

After having carried out the translatory relative motion between the cartridge assembly 301c and the housing assembly 301h, the user now rotates the needle cap body 331 in the direction of the arrow seen in Fig. 30. This causes the abutment surfaces 332s to interact with the respective reaction surfaces 357, and the lock ring 350 resultantly rotates jointly with the needle cap body 331. Since the stator 360 is rotationally fixed in the outer housing and the cartridge flanges 316 are positioned in the openings 365, the cartridge 310 remains stationary relative to the outer housing. The rotation of the lock ring 350 causes an angular displacement of the shelves 356, as well as of the hanger profile due to the arms 326 being received in the bores 355.

During the rotation the arms 326 travel the curved slots 366 from end to end, and the circumferential extent of the curved slots 366 thus defines the possible angular displacement of the needle cap body 331 relative to the outer housing. At the point when the needle cap body 331 meets the rotational stop the shelves 356 have moved to a position just below the cartridge flanges 316, and the lock ring 350 thus supports and prevents axial movement of the cartridge 310. This can be seen from Fig. 31.

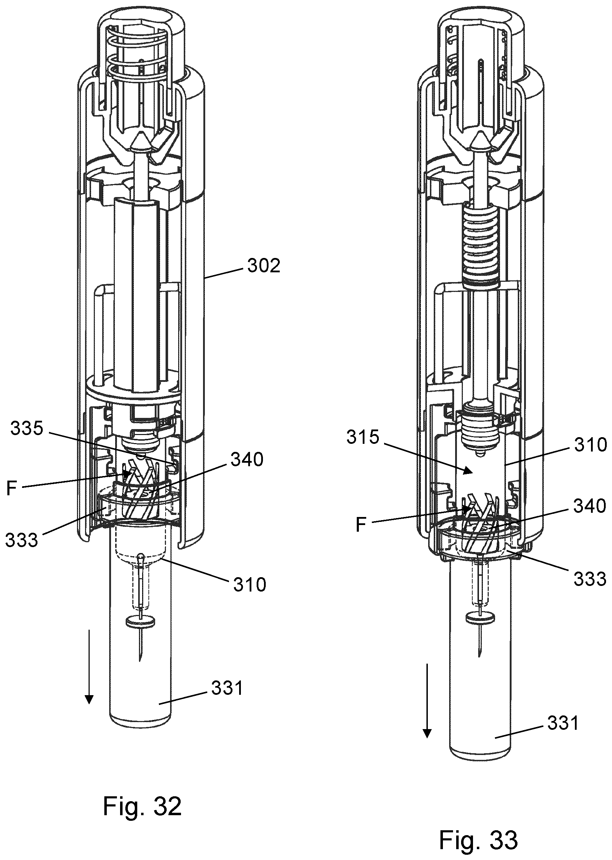

With the cartridge 310 now in position and fixed with respect to the outer housing, the user pulls the needle cap body 331 away from the housing assembly 301h, in the direction of the arrow shown in Fig. 32. This introduces an axial motion of the overhang 333 relative to the cartridge 310 which due to the magnet 334 held in the overhang 333 causes a distal pull on the magnetic mixer element 340. The magnetic mixer element 340 consequently moves downwards in the interior 315 of the cartridge 310 which forces the liquid in the front portion of the interior 315 to pass the body 341 partially through the bore 345 and partially through the inclined canals 344, generating a high-speed swirling motion of the liquid in the wake of the body 341, as indicated by the streamlines, F, in Figs. 32 and 33.

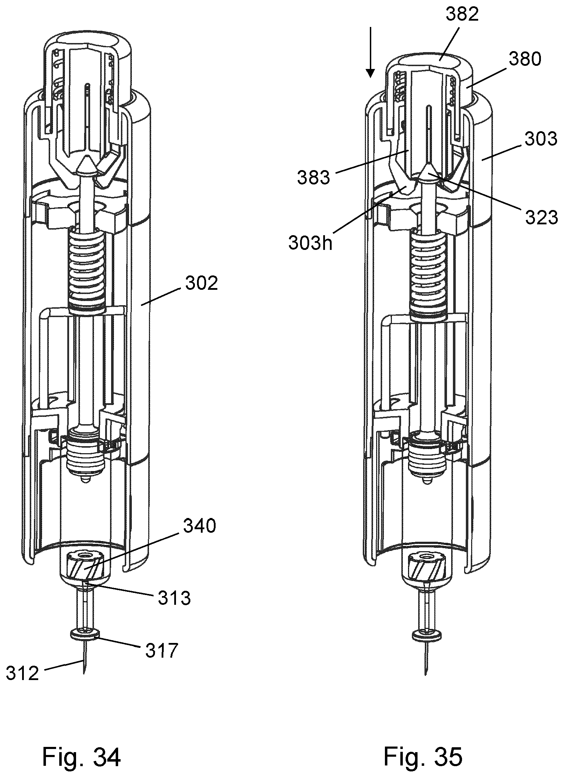

Hence, when the needle cap body 331 has been pulled completely out of the distal housing part 304 the magnetic mixer element 340 resides at the outlet end portion 311 and the pharmaceutical suspension is in re-suspended form and ready for dose administration. In Fig. 34 the exposed injection needle 312 has been inserted through a skin barrier (not shown), and the outlet end portion 311 has been pressed down against the disc 317, whereby a rear portion of the injection needle 312 has slid into the interior 315 of the cartridge 310, causing penetration of the septum 313 (not visible).

To perform a dose administration the user now depresses the push button 380 in the direction of the arrow shown in Fig. 35. The downward movement of the push button 380 will eventually cause the stalk 383 to engage and radially deflect the flexible fingers 303h which consequently disengage from the stud 323.

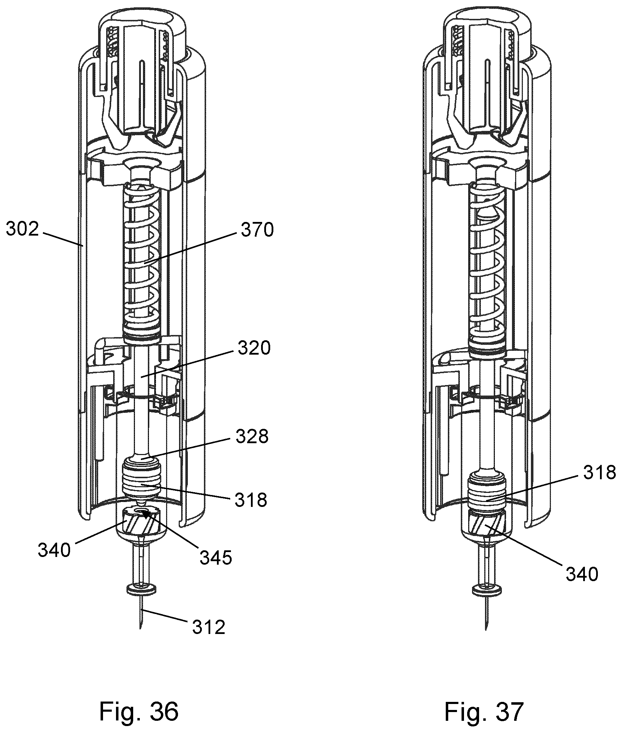

As seen in Fig. 36 this releases the drive spring 370 which expands and forces the distal spring base 371 and thereby the piston rod 320 downwards. The piston rod foot 328 resultantly advances the piston 318 distally in the interior 315 of the cartridge 310 and the re-suspended pharmaceutical suspension is thus expelled through the bore 345 and the injection needle 312 into the skin.

In Fig. 37 the piston 318 abuts the magnetic mixer element 340 and the cartridge 310 is (substantially) emptied. The user can therefore retract the injection needle 312 from the skin, re-insert the needle cap body 331 linearly into the housing assembly 301h and remove the used cartridge 310 from the lock ring 350 by rotating the needle cap body 331 reversely to the rotational direction during attachment of the cartridge 310 and pulling the needle cap body 331 with the cartridge 310 therein away from the distal housing part 304. During the proximal motion of the needle cap body 331 relative to the outer housing the cartridge flanges 332 abut the arms 326 and lift the piston rod 320 upwards until the stud 323 meets and snaps in behind the flexible fingers 303h, whereby the drive spring 370 becomes re-cocked. The housing assembly 301h is thereby readied for use with a new cartridge assembly.

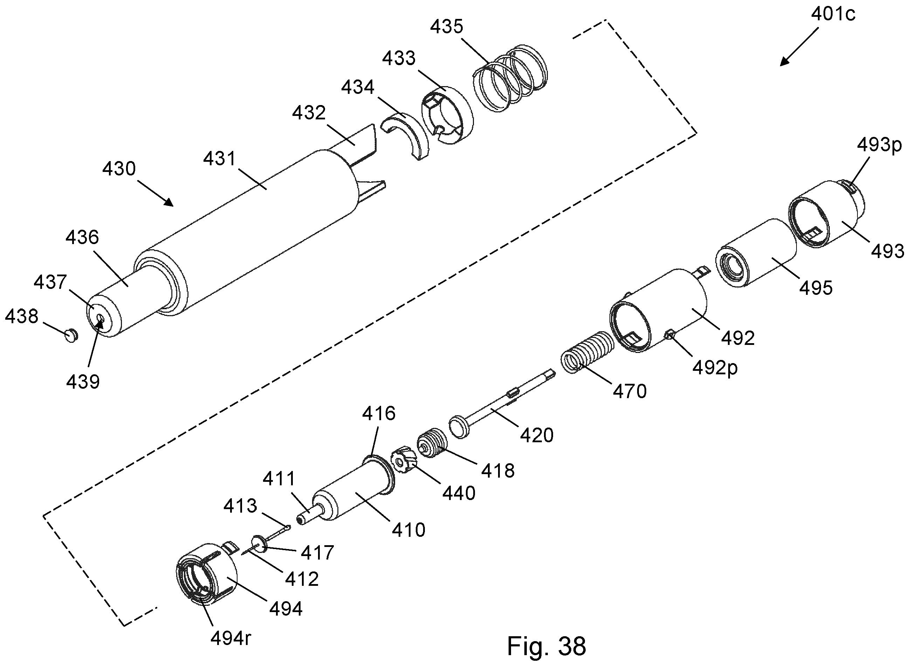

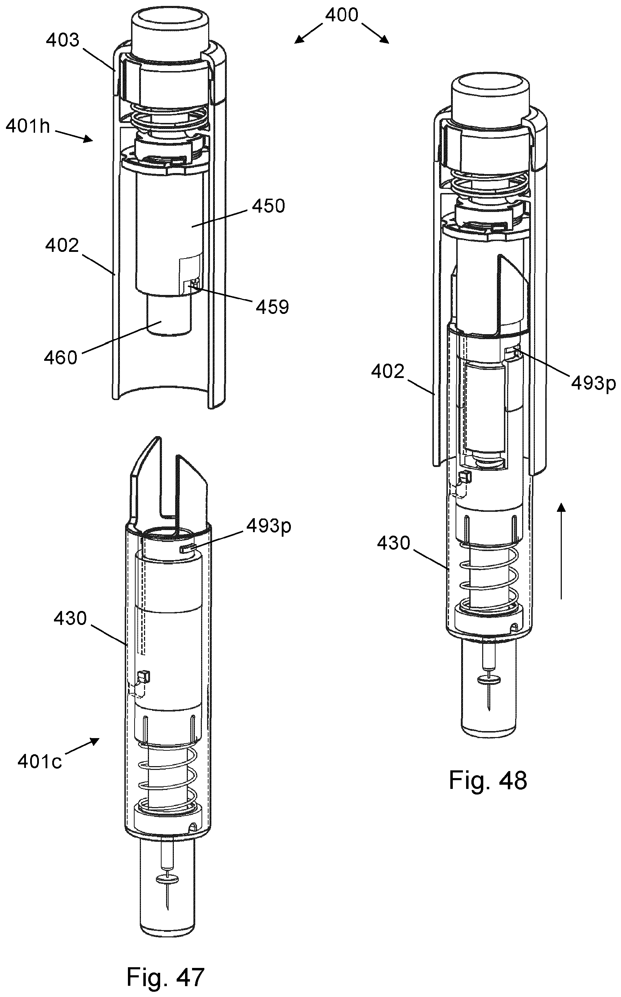

Fig. 38 is an exploded view of a cartridge assembly 401c forming part of a dose delivery device 400 (see Fig. 47) according to a fifth exemplary embodiment of the invention. The cartridge assembly 401c comprises a cartridge 410 extending along a reference axis and having a distal outlet portion 411. The cartridge 410 holds a volume of a pharmaceutical suspension (not visible) and a magnetic mixer element 440, similar to the magnetic mixer element 340 described above in connection with the fourth exemplary embodiment of the invention.

The cartridge 410 is sealed proximally by a slidable piston 418 and distally by a penetrable septum 413 disposed about a rear portion of an injection needle 412 and fixed to the outlet portion 411. The penetrable septum 413 may e.g. comprise an elastomeric needle coating applied directly to the injection needle 412 such that an initial adhesion between the elastomeric needle coating and the injection needle 412 is provided, which initial adhesion is irreversibly breakable by relative axial displacement between the two. A disc 417 is fixedly mounted on the injection needle 412 at a position which defines the possible depth of insertion of the injection needle 412 in the skin. At its proximal end the cartridge 410 is provided with a flange 416.

The cartridge assembly 401c further comprises a three-part inner housing consisting of a central inner housing part 492, a proximal inner housing part 493 being snap-fitted to a proximal end portion of the central inner housing part 492, and a distal inner housing part 494 being snap-fitted to a distal end portion of the central inner housing part 492. The proximal inner housing part 493 has a pair of radially opposite proximal protrusions 493p (only one is visible in Fig. 38), while the central inner housing part 492 has a pair of radially opposite central protrusions 492p. The distal inner housing part 494 has a receiving section 494r at its distal end configured to receive and retain the flange 416, whereby the cartridge 410 is axially fixed with respect to the inner housing.

The inner housing is configured to accommodate a rotor 495, a piston rod 420 adapted to drive the piston 418, and a drive spring 470 capable of storing energy and releasing stored energy to actuate the piston rod 420.

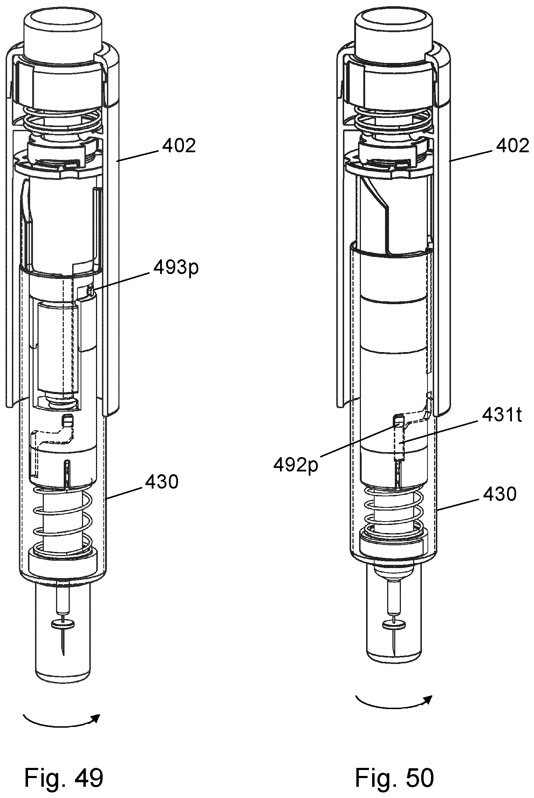

The cartridge 410 and the inner housing are arranged within a shield member 430 which comprises a tubular shield body 431, a distal needle shield portion 436, and a pair of proximally extending arms 432. The shield body 431 has two diametrically opposite interior tracks 431t (one is visible in Fig. 50) along inner surface portions, configured to slidingly engage with the central protrusions 492p on the central inner housing part 492. The distal needle shield portion 436 has a transversal end wall 437 with an opening 439 in which a penetrable shield seal 438 is arranged. The shield member 430 is biased by a shield spring 435 and carries a semi-circular magnet 434 which is retained in a magnet holder 433 arranged at the distal end of the shield body 431.

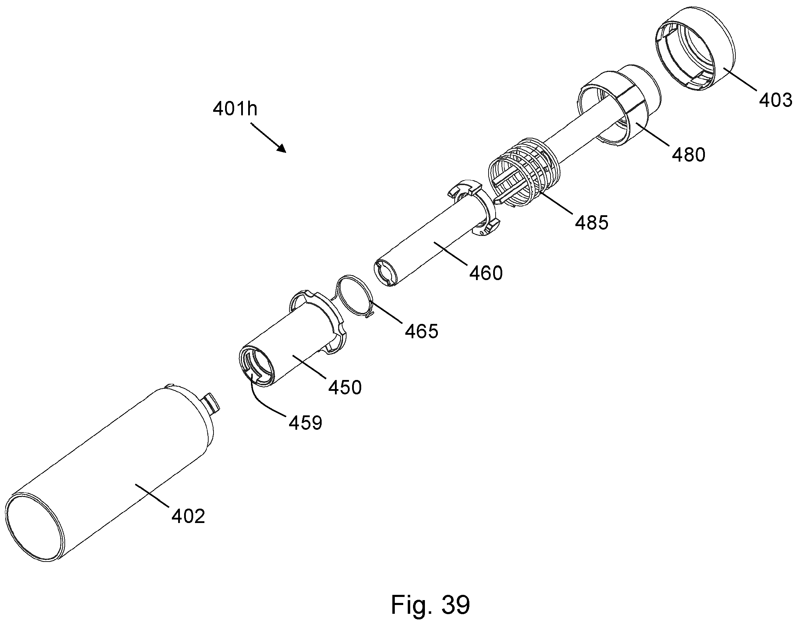

Fig. 39 is an exploded view of a housing assembly 401h of the dose delivery device 400. The housing assembly 401h comprises a main housing part 402 and a top housing part 403 snap-fitted thereto, a tubular base member 450 which at a distal end portion is provided with a pair of diametrically opposite inner bayonet tracks 459 (only one is visible), a lock member 460, a lock spring 465, a dose release button 480, a proximal portion of which extends through a proximal opening in the top housing part 403, and a button spring 485. The base member 450 is accommodated by, and axially and rotationally fixed to, the main housing part 402.

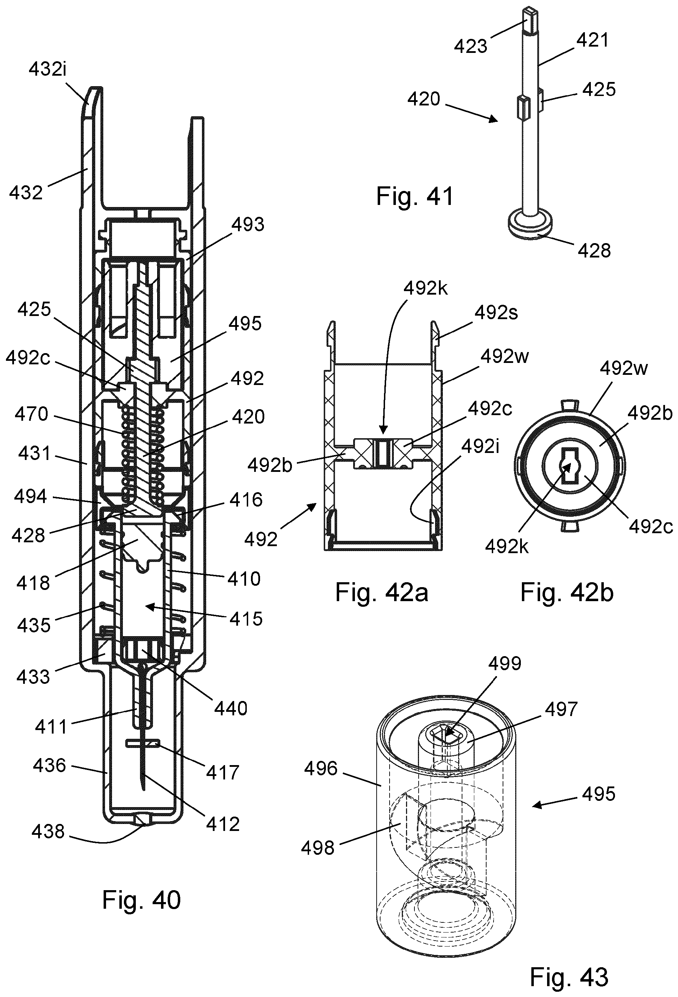

Fig. 40 is a longitudinal section view of the cartridge assembly 401c, showing the relative positions of the constituent components in a pre-use state and further detailing structural features of some of these components. As can be seen, each of the proximally extending arms 432 of the shield member 430 are provided with an inclined proximal end 432i. Furthermore, the piston rod 420 has a distal piston rod foot 428 adapted to abut a proximal surface of the piston 418 and acting as a distal base for the drive spring 470, as well as a pair of radial protuberances 425 which in the pre-use state abut a proximal side of a central barrier 492c in an interior of the central inner housing part 492. A distal side of the central barrier 492c acts as a proximal base for the drive spring 470 which is thus, in this state of the cartridge assembly 401c, pre-tensioned and safely cocked because the piston rod 428 is unable to move distally relative to the inner housing. It is noted that the magnetic mixer element 440 is initially positioned distally in a cartridge interior 415, surrounded partially by the semi-circular magnet 434 in the magnet holder 433.

Fig. 41 is a perspective view of the piston rod 420, which in addition to the piston rod foot 428 and the radial protuberances 425 comprises a circular-cylindrical main piston rod body 421 and a proximal end piece 423 with a rectangular cross-section.

Fig. 42a is a longitudinal section view of the central inner housing part 492, which comprises a cylindrical wall 492w, a pair of proximal snap arms 492s for interlocking engagement with the proximal inner housing part 493, and a pair of distal indentations 492i for interlocking engagement with the distal inner housing part 494. The central barrier 492c has a through-going keyhole 492k and is connected to the cylindrical wall 492w by an annular bridge section 492b. The keyhole 492k has a configuration similar to, but slightly bigger than, the cross-section of the main piston rod body 421 with the radial protuberances 425, and the central barrier 492c thus allows passage of the radial protuberances 425 only at a certain angular orientation of the piston rod 420 relative to the central inner housing part 492. The configuration of the keyhole 492k can be seen in Fig. 42b, which is a top view of the central inner housing part 492.

Fig. 43 is a perspective view of the rotor 495 which has a circular-cylindrical outer wall 496 that for the sake of clarity is shown as transparent to visualise the interior contours (depicted with dotted lines). The rotor 495 is formed with an interior tower 497 adapted to accommodate a proximal portion of the piston rod 420. Hence, the tower 497 defines a narrow space which is shaped substantially like that proximal portion of the piston rod 420. In particular, the tower 497 comprises a proximal portion having an opening 499 which is rectangular in cross-section and which mates with the proximal end piece 423 to provide for a rotationally interlocked, but axially free, connection between the piston rod 420 and the rotor 495. A couple of spiralling ramps 498 are formed between the outer wall 496 and the tower 497. The ramps 498 extend approximately half a revolution and are 180º offset from one another.

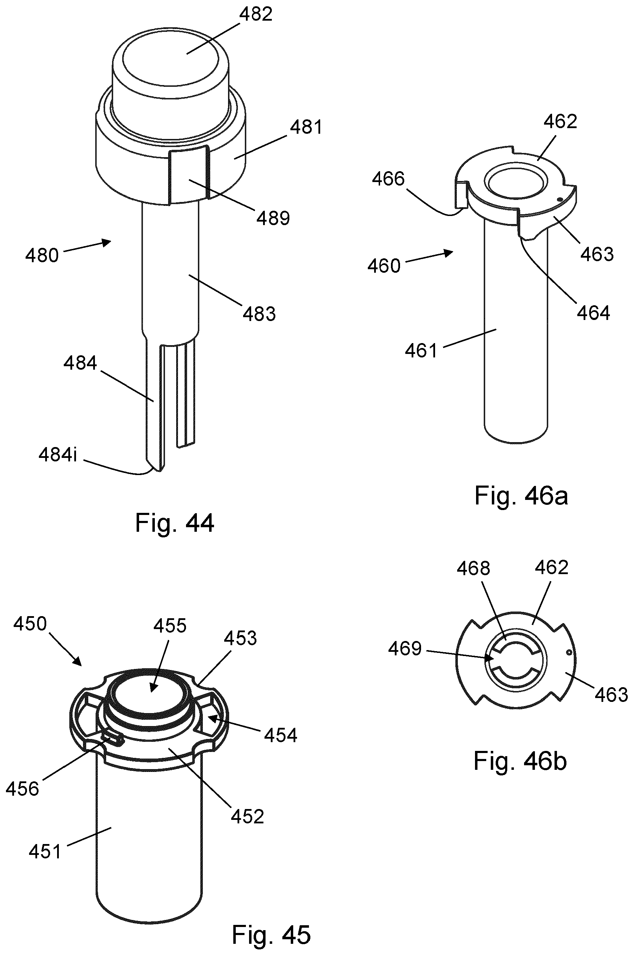

Fig. 44 is a perspective view of the dose release button 480, which comprises a cylindrical body 483 with two legs 484, each having an inclined distal end surface 484i, a proximal collar portion 481, and a slightly concave top surface 482. The collar portion 481 is provided with three circumferentially equidistantly spaced carvings 489 (only one is visible) which are configured for sliding engagement with mating protrusions on an interior surface of the top housing part 403 to rotationally lock the dose release button 480 with respect to the top housing part 403 and the main housing part 402.

Fig 45 is a perspective view of the base member 450, which comprises a base member body 451 with a through-going bore 455, a base member flange 452, which is provided with four circumferentially spaced apart notches 453 configured for engagement with protrusions on an inner surface of the main housing part 402, a stud 456 defining an initial angular position of the lock member 460 relative to the base member 450, and two diametrically opposite slots 454 configured to allow sliding reception of the proximally extending arms 432, the base member 450 and the shield member 430 thereby being rotationally interlocked but allowed to undergo relative axial motion.

Figs. 46a and 46b are respectively a perspective view and a top view of the lock member 460, which comprises a lock member body 461 and a proximal lock member flange 462. The lock member flange 462 has two diametrically opposite radial protrusions 463, each having an inclined edge portion 464 configured for interaction with one of the inclined proximal ends 432i of the proximally extending arms 432 and a distal surface portion 466 for abutment with the base member flange 452.

The distal end of the lock member body 461 is provided with two inwardly projecting ledges 468, spaced apart to form diametrically opposite gaps 469 therebetween. The gaps 469 allow for passage of the legs 484 when the lock member 460 and the dose release button 480 are in a certain relative angular position.

In the housing assembly 401h the lock member body 461 extends through the bore 455 and the distal surface portions 466 rest on a proximal face of the base member flange 452. The lock member 460 is thus axially restricted in the main housing part 402 but capable of rotation relative thereto. The lock spring 465 is a torsion spring which biases one of the radial protrusions 463 into abutment with the stud 456.

In the following, an assembly and use of the dose delivery device 400 will be described with reference to Figs. 47-64.

Fig. 47 is a perspective view of the dose delivery device 400 before attachment of the cartridge assembly 401c to the housing assembly 401h. For the sake of clarity, a portion of the main housing part 402 has been cut away, and a portion of the base member body 451 as well as the entire shield member 430 are shown as transparent.

To assemble the dose delivery device 400, the cartridge assembly 401c is firstly moved linearly in the proximal direction, as indicated by the arrow in Fig. 48, until the proximal protrusions 493p reach an end of the entrance sections of the bayonet tracks 459. After this, the shield member 430 is turned counter-clockwise relative to the main housing part 402 to allow the proximal protrusions 493p to travel to the end of the bayonet tracks 459 and thereby axially lock the inner housing and the cartridge 410 with respect to the base member 450 and the main housing part 402. This is indicated in Fig. 49.

Further counter-clockwise rotation of the shield member 430 relative to the main housing part 402, as depicted in Fig. 50, now causes a relative angular displacement between the shield body 431 and the inner housing because the proximal inner housing part 493 is prevented from further counter-clockwise rotation relative to the base member 450 due to the position of the proximal protrusions 493p at the ends of the bayonet tracks 459. As a result, the central protrusions 492p are forced to travel respective circumferential track portions of the interior tracks 431t and align with connected axial track portions, thereby rendering the shield member 430 axially displaceable relative to the inner housing. The dose delivery device 400 is now in an unlocked state, ready for an injection action.

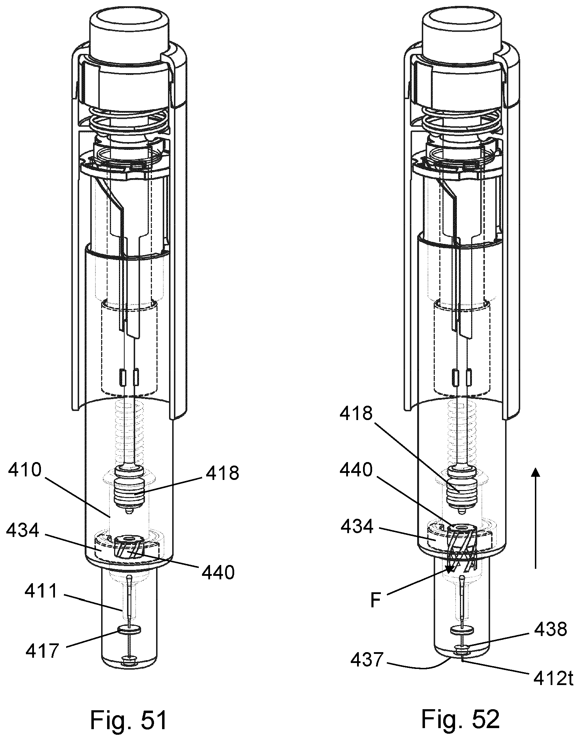

Figs. 51-54 show the dose delivery device 400 in a further stripped-down version, where some elements have been removed and some made transparent (some contours are indicated with dotted lines) to enable a visualisation of what happens inside the cartridge 410. The state of the dose delivery device 400 shown in Fig 51 thus corresponds to that shown in Fig. 50. So, in the unlocked state of the dose delivery device 400 the magnetic mixer element 440 is positioned axially opposite to the piston 418, in a distal portion of the cartridge 410, and the disc 417 is axially spaced apart from the outlet portion 411.

To insert the injection needle 412 the user places the transversal end wall 437 at a desired place on the skin and presses the main housing part 402 towards the body. As shown in Fig. 52, this causes a front tip 412t of the injection needle 412 to penetrate the shield seal 438 as the shield member 430 is resultantly displaced proximally relative to the main housing part 402. Also, due to the proximal displacement of the shield member 430 relative to the main housing part 402 the magnet 434 moves proximally relative to the cartridge 410, whereby the magnetic mixer element 440 is forced to slide upwards in the cartridge interior 415, generating a swirling motion of the pharmaceutical suspension in its wake, as indicated by streamlines, F.

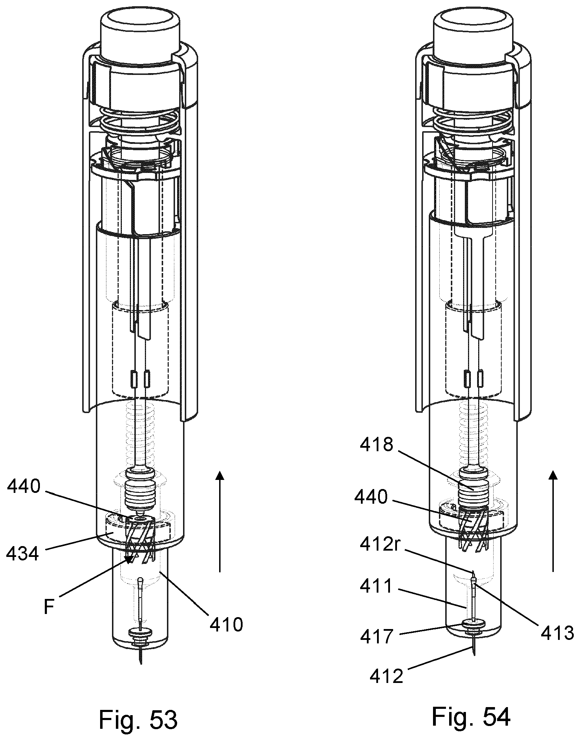

As the main housing part 402 comes gradually closer to the body of the user, and the shield member 430 thereby is pressed further proximally into the main housing part 402, the injection needle 412 is inserted deeper into the skin, until the transversal end wall 437 with the shield seal 438 reaches the disc 417 as shown in Fig. 53, and the magnetic mixer element 440 is moved further upwards in the cartridge interior 415, stirring up more of the pharmaceutical suspension.

When the transversal end wall 437 has reached the disc 417, further movement of the main housing part 402 towards the body causes a relative converging motion between the cartridge 410 and the disc 417 as the outlet portion 411 is forced into contact with the disc 417. During this converging motion the initial adhesion between the penetrable septum 413, being fixed to the outlet portion 411 and the injection needle 412, on which the disc 417 is fixedly mounted, breaks, and a rear tip 412r of the injection needle 412 resultantly transpierces the penetrable septum 413 as the injection needle 412 slides a small distance into the cartridge interior 415. This is seen from Fig. 54.

The shield member 430 is now fully depressed into the main housing part 402, the magnetic mixer element 440 has travelled the entire cartridge interior 415 and reached the piston 418, thereby ensuring complete re-suspension of the pharmaceutical suspension, and fluid communication between the cartridge interior 415 and the injection needle 412, partially residing in the body of the user, is established.

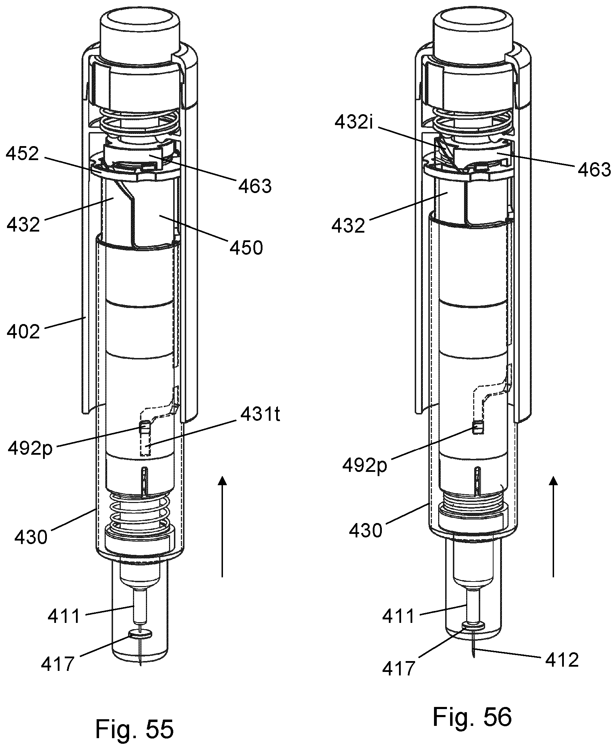

The state changes of the dose delivery device 400 described in connection with Figs. 51-54 also comprise a number of other notable component movements. Figs. 55 and 56 show the dose delivery device 400 with a non-transparent inner housing so as to allow a description of what happens in a layer exteriorly of the cartridge 410. The state of the dose delivery device 400 shown in Fig. 55 corresponds to the state shown in Fig. 52, and the state of the dose delivery device 400 shown in Fig. 56 corresponds to the state shown in Fig. 54.

Hence, in Fig. 55 the shield member 430 has moved slightly proximally relative to the main housing part 402, allowing the front tip 412t to penetrate the shield seal 438 (not visible in Fig. 55). The disc 417 is still axially spaced apart from the outlet portion 411 at this point. The axial movement of the shield member 430 is possible because the central protrusions 492p are guided in the axial track portions of the interior tracks 431t. As the shield body 431 thus translates proximally relative to the base member 450 the proximally extending arms 432 extend through the slots 454 in the base member flange 452. Proximally of the base member flange 452 the inclined proximal ends 432i interface with the radial protrusions 463, and as the proximally extending arms 432 continue the proximal motion this interfacing leads to the inclined edge portions 464 sliding along the inclined proximal ends 432i and thereby to a rotation of the lock member 460 about the central axis of the main housing part 402. The rotation of the lock member 460 continues until the central protrusions 492p reach the respective ends of the axial track portions of the interior tracks 431t, as seen in Fig. 56. This is the point where the shield member 430 is fully depressed into the main housing part 402.

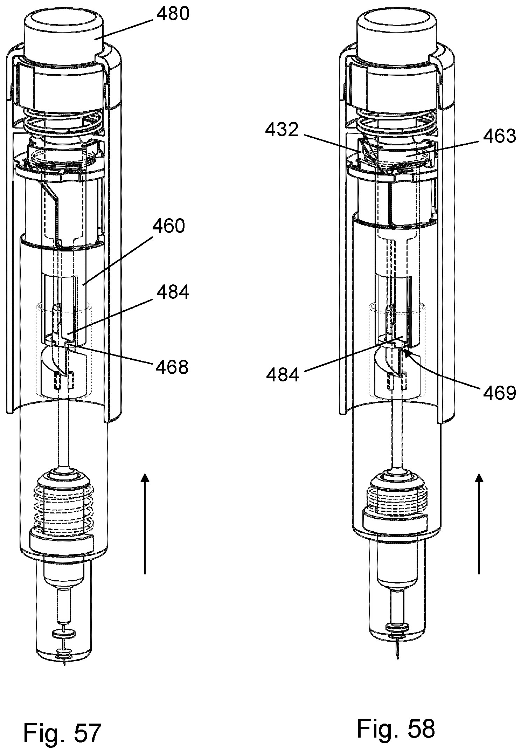

Figs. 57 and 58 show the dose delivery device 400 without the inner housing and with a transparent outer wall 496 of the rotor 495 and a transparent lock member 460 to allow a description of the component movements in a further layer of the construction. The state of the dose delivery device 400 shown in Fig. 57 corresponds to the state shown in Figs. 52 and 55, and the state of the dose delivery device 400 shown in Fig. 58 corresponds to the state shown in Figs. 54 and 56.

Fig. 57 shows that the legs 484 initially rest on the ledges 468 of the lock member 460 and that the dose release button 480 thereby is prevented from distal movement relative to the main housing part 402. However, the above-described proximal displacement of the shield member 430 which leads to the rotation of the lock member 460 causes the lock member 460 to eventually take up an angular position relative to the dose release button 480 in which the gaps 469 are aligned with the legs 484. In this particular relative angular position, shown in Fig. 58, the dose release button 480 is no longer prevented from distal movement relative to the main housing part 402, and an activation of the injection mechanism is thus now enabled.

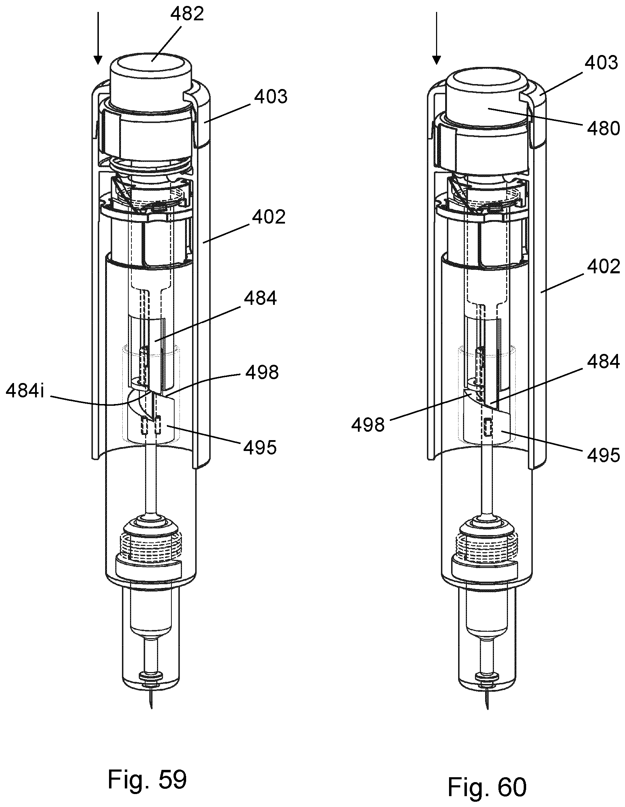

With the injection needle 412 safely inserted into the skin the user depresses the top surface 482 towards the top housing part 403 to perform an injection. As shown in Fig. 59 this initially causes the inclined distal end surfaces 484i of the legs 484 to move through the gaps 469 and into sliding abutment with the spiralling ramps 498. As the dose release button 480 is depressed further into the main housing part 402 the legs 484 travel along the ramps 498, causing the rotor 495 to rotate about the central axis. From Fig. 60 it is clear that the rotor 495 thus rotates clockwise about the central axis (when seen from a proximal perspective).

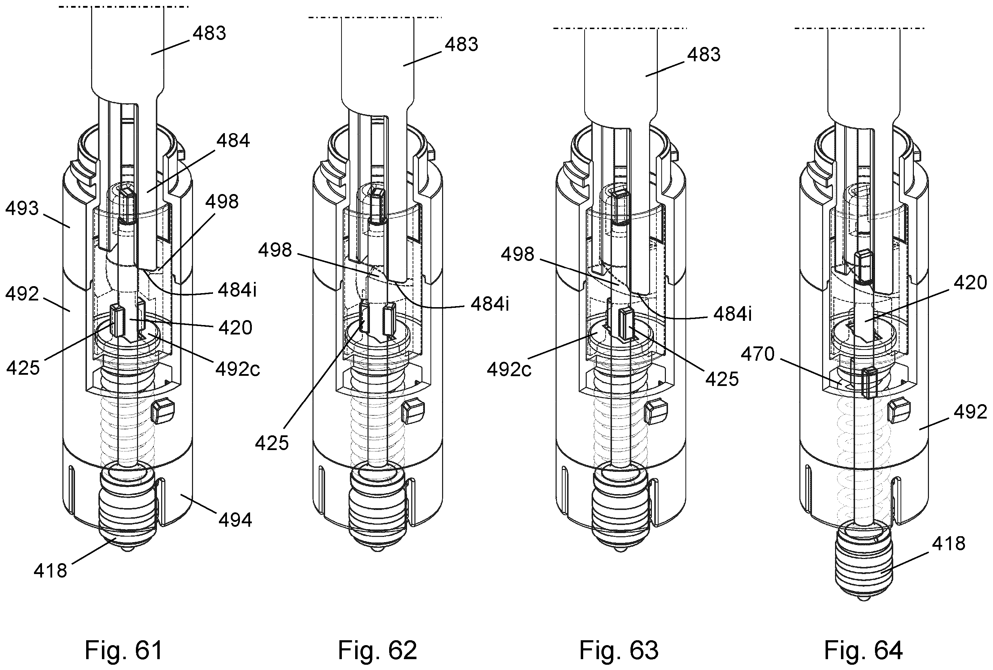

Because of the mating connection between the opening 499 and the proximal end piece 423 the rotation of the rotor 495 causes a like rotation of the piston rod 420. Figs. 61-63 show the resulting angular displacement of the piston rod 420 as the legs 484 travel down the ramps 498 (depicted in dotted lines). Initially (Fig. 61), the main piston rod body 421 is oriented such that the radial protuberances 425 rest on the central barrier 492c, immobilising the piston rod 420, and eventually (Fig. 63), the main piston rod body 421 is turned 90º clockwise relative to the central inner housing part 492, resulting in the radial protuberances 425 becoming properly aligned with the keyhole 492k.

When the radial protuberances 425 and the keyhole 492k are so aligned the drive spring 470 is allowed to expand, and the energy released by the expansion of the drive spring 470 will urge the piston rod 420 downwards through the tower 497, whereby the piston 418 and the magnetic mixing element 440 are forced distally in the cartridge interior 415 towards the outlet portion 411 to eject a predetermined dose of the re-suspended pharmaceutical suspension through the injection needle 412. This is indicated in Fig. 64.

## Claims

1. A dose delivery device (1, 100, 200, 300, 400) comprising:
- a variable volume reservoir (10, 110, 210, 310, 410) holding a pharmaceutical suspension and comprising an outlet (12, 112, 212, 312, 412),
- a dose expelling mechanism adapted for activation to expel a volume of the pharmaceutical suspension through the outlet (12, 112, 212, 312, 412), and
- a dose preparation system comprising a preparation member (30, 130, 230, 330, 430) operable prior to activation of the dose expelling mechanism to enable administration of the volume of the pharmaceutical suspension to a subject,
wherein the dose preparation system further comprises an agitation member (40, 140, 240, 340, 440) capable of agitating relative motion with respect to the variable volume reservoir (10, 110, 210, 310, 410), the agitating relative motion causing a re-suspending agitation of the pharmaceutical suspension, and
wherein the agitation member (40, 140, 240, 340, 440) is operatively coupled with the preparation member (30, 130, 230, 330, 430) and configured to undergo said agitating relative motion in response to the operation of the preparation member (30, 130, 230, 330, 430)
**characterised in that** the variable volume reservoir (10, 110) is a flexible foil reservoir and the agitation member (40, 140) comprises a deformation element (44a, 144a) adapted to deform the flexible foil reservoir to thereby provoke the re-suspending agitation of the pharmaceutical suspension, the deformation element (44a, 144a) being adapted to deform the flexible foil reservoir by sweeping and squeezing an outer surface thereof.

2. A dose delivery device according to claim 1, wherein the agitating relative motion comprises a movement of the agitation member (40, 140, 240, 340, 440) relative to the variable volume reservoir (10, 110, 210, 310, 410) from a first predetermined position to a second predetermined position.

3. A dose delivery device according to claim 1 or 2, further comprising a releasable lock (143, 460) switchable from an initial state in which activation of the dose expelling mechanism is prevented to a released state in which activation of the dose expelling mechanism is enabled, the releasable lock (143, 460) being operatively coupled with the preparation member (130, 430) and configured to switch from the initial state to the released state in response to the operation of the preparation member (130, 430).

4. A dose delivery device according to any of the preceding claims, further comprising a housing (2, 3) accommodating at least a portion of the dose expelling mechanism and defining a reference axis,
wherein the preparation member (30) comprises a cap removably attached to the housing (2, 3) to cover the outlet (12),
wherein the deformation element (44a) is attached to, or forms part of, the cap, and wherein the cap is adapted to be removed by relative axial motion with respect to the housing (2, 3) and the flexible foil reservoir, the deformation element (44a) thereby sweeping and squeezing the outer surface of the flexible foil reservoir.

5. A dose delivery device according to claim 4, wherein the agitation member (40) further comprises a second deformation element (44b) and a third deformation element (44c) arranged axially spaced apart from one another and from the deformation element (44a), and wherein at least two of the deformation elements (44a, 44b, 44c) intersect the reference axis at different angles.

6. A dose delivery device according to any of claims 1 to 3, further comprising a housing (102, 103) accommodating at least a portion of the dose expelling mechanism and defining a reference axis,
wherein the preparation member (130) comprises a pull tab removably attached to the housing (102, 103),
wherein the deformation element (144a) is attached to, or forms part of, the pull tab, and
wherein the pull tab is adapted to be removed by relative transversal motion with respect to the housing (102, 103) and the flexible foil reservoir, the deformation element (144a) thereby sweeping and squeezing the outer surface of the flexible foil reservoir.

7. A dose delivery device according to claim 6, wherein the agitation member (140) further comprises a second deformation element (144b) arranged transversally spaced apart from the deformation element (144a), and
wherein the two deformation elements (144a, 144b) intersect the reference axis at different angles.

8. A dose delivery device according to claim 6 or 7, further comprising a cap (150) removably attached to the housing (102, 103) to cover the outlet (112),
wherein the agitation member (140) and the cap (150) comprise mutually interacting contact members (143, 152) configured to prevent removal of the cap (150) when the pull tab is attached to the housing (102, 103).

9. A dose delivery device according to any of claims 6 - 8, wherein the dose expelling mechanism comprises an actuator (120) and a compression member (127, 128) adapted to collapse the flexible foil reservoir in response to an axial displacement of the actuator (120) relative to the housing (102, 103) from a first axial position to a second axial position, and wherein the agitation member (140) is configured to block movement of the actuator (120) from the first axial position towards the second axial position when the pull tab is attached to the housing (102, 103).

## Patentansprüche

1. Dosisabgabevorrichtung (1, 100, 200, 300, 400), umfassend:
- ein Reservoir (10, 110, 210, 310, 410) mit variablem Volumen, das eine pharmazeutische Suspension enthält und einen Auslass (12, 112, 212, 312, 412) umfasst,
- einen Dosisausstoßmechanismus, der zur Aktivierung angepasst ist, um ein Volumen der pharmazeutischen Suspension durch den Auslass (12, 112, 212, 312, 412) auszustoßen, und
- ein Dosisvorbereitungssystem, das ein Vorbereitungselement (30, 130, 230, 330, 430) umfasst, das vor der Aktivierung des Dosisausstoßmechanismus betätigbar ist, um die Verabreichung des Volumens der pharmazeutischen Suspension an ein Subjekt zu ermöglichen,
wobei das Dosisvorbereitungssystem ferner ein Rührelement (40, 140, 240, 340, 440) umfasst, das zu einer relativen Rührbewegung in Bezug auf das Reservoir (10, 110, 210, 310, 410) mit variablem Volumen in der Lage ist, wobei die relative Rührbewegung ein resuspendierendes Rühren der pharmazeutischen Suspension bewirkt, und
wobei das Rührelement (40, 140, 240, 340, 440) mit dem Vorbereitungselement (30, 130, 230, 330, 430) betriebsmäßig gekoppelt ist und konfiguriert ist, die relative Rührbewegung als Reaktion auf den Betrieb des Vorbereitungselements (30, 130, 230, 330, 430) zu durchlaufen,
**dadurch gekennzeichnet, dass** das Reservoir (10, 110) mit variablem Volumen ein Reservoir mit flexibler Folie ist und das Rührelement (40, 140) ein Verformungselement (44a, 144a) umfasst, das angepasst ist, um das Reservoir mit flexibler Folie zu verformen, wodurch das resuspendierende Rühren der pharmazeutischen Suspension hervorgerufen wird, wobei das Verformungselement (44a, 144a) angepasst ist, um das Reservoir mit flexibler Folie durch Überstreichen und Zusammendrücken einer äußeren Oberfläche davon zu verformen.

2. Dosisabgabevorrichtung nach Anspruch 1, wobei die relative Rührbewegung eine Bewegung des Rührelements (40, 140, 240, 340, 440) relativ zu dem Reservoir (10, 110, 210, 310, 410) mit variablem Volumen von einer ersten vorbestimmten Position zu einer zweiten vorbestimmten Position umfasst.

3. Dosisabgabevorrichtung nach Anspruch 1 oder 2, ferner umfassend eine lösbare Verriegelung (143, 460), die von einem Ausgangszustand, in dem die Aktivierung des Dosisausstoßmechanismus verhindert wird, in einen freigegebenen Zustand, in dem die Aktivierung des Dosisausstoßmechanismus ermöglicht wird, umschaltbar ist, wobei die lösbare Verriegelung (143, 460) mit dem Vorbereitungselement (130, 430) betriebsmäßig gekoppelt und so konfiguriert ist, dass sie als Reaktion auf den Betrieb des Vorbereitungselements (130, 430) von dem Ausgangszustand in den freigegebenen Zustand umschaltet.

4. Dosisabgabevorrichtung nach einem der vorhergehenden Ansprüche, ferner umfassend ein Gehäuse (2, 3), das mindestens einen Teil des Dosisausstoßmechanismus aufnimmt und eine Bezugsachse definiert,
wobei das Vorbereitungselement (30) eine Kappe umfasst, die abnehmbar an dem Gehäuse (2, 3) angebracht ist, um den Auslass (12) abzudecken,
wobei das Verformungselement (44a) an der Kappe angebracht ist oder einen Teil davon bildet, und
wobei die Kappe angepasst ist, um durch relative axiale Bewegung in Bezug auf das Gehäuse (2, 3) und das Reservoir mit flexibler Folie entfernt zu werden, wobei das Verformungselement (44a) dadurch die äußere Oberfläche des Reservoirs mit flexibler Folie überstreicht und zusammendrückt.

5. Dosisabgabevorrichtung nach Anspruch 4, wobei das Rührelement (40) ferner ein zweites Verformungselement (44b) und ein drittes Verformungselement (44c) umfasst, die axial voneinander und von dem Verformungselement (44a) beabstandet angeordnet sind, und
wobei mindestens zwei der Verformungselemente (44a, 44b, 44c) die Bezugsachse unter unterschiedlichen Winkeln schneiden.

6. Dosisabgabevorrichtung nach einem der Ansprüche 1 bis 3, ferner umfassend ein Gehäuse (102, 103), das mindestens einen Teil des Dosisausstoßmechanismus aufnimmt und eine Bezugsachse definiert,
wobei das Vorbereitungselement (130) eine Zuglasche umfasst, die abnehmbar an dem Gehäuse (102, 103) angebracht ist,
wobei das Verformungselement (144a) an der Zuglasche angebracht ist oder einen Teil davon bildet, und
wobei die Zuglasche angepasst ist, um durch relative Querbewegung in Bezug auf das Gehäuse (102, 103) und das Reservoir mit flexibler Folie entfernt zu werden, wobei das Verformungselement (144a) dadurch die äußere Oberfläche des Reservoirs mit flexibler Folie überstreicht und zusammendrückt.

7. Dosisabgabevorrichtung nach Anspruch 6, wobei das Rührelement (140) ferner ein zweites Verformungselement (144b) umfasst, das quer beabstandet von dem Verformungselement (144a) angeordnet ist, und
wobei die zwei Verformungselemente (144a, 144b) die Bezugsachse unter unterschiedlichen Winkeln schneiden.

8. Dosisabgabevorrichtung nach Anspruch 6 oder 7, ferner umfassend eine Kappe (150), die abnehmbar an dem Gehäuse (102, 103) angebracht ist, um den Auslass (112) abzudecken,
wobei das Rührelement (140) und die Kappe (150) miteinander zusammenwirkende Kontaktelemente (143, 152) umfassen, die so konfiguriert sind, dass sie ein Entfernen der Kappe (150) verhindern, wenn die Zuglasche an dem Gehäuse (102, 103) angebracht ist.

9. Dosisabgabevorrichtung nach einem der Ansprüche 6 bis 8, wobei der Dosisausstoßmechanismus ein Betätigungselement (120) und ein Kompressionselement (127, 128) umfasst, das angepasst ist, um das Reservoir mit flexibler Folie in Reaktion auf eine axiale Verschiebung des Betätigungselements (120) relativ zu dem Gehäuse (102, 103) von einer ersten axialen Position zu einer zweiten axialen Position zusammenzuklappen, und wobei das Rührelement (140) so konfiguriert ist, dass es eine Bewegung des Betätigungselements (120) von der ersten axialen Position in Richtung der zweiten axialen Position blockiert, wenn die Zuglasche an dem Gehäuse (102, 103) angebracht ist.

## Revendications

1. Dispositif de distribution de dose (1, 100, 200, 300, 400) comprenant :
- un réservoir à volume variable (10, 110, 210, 310, 410) contenant une suspension pharmaceutique et comprenant une sortie (12, 112, 212, 312, 412),
- un mécanisme d'expulsion de dose adapté pour une activation afin d'expulser un volume de la suspension pharmaceutique à travers la sortie (12, 112, 212, 312, 412), et
- un système de préparation de dose comprenant un élément de préparation (30, 130, 230, 330, 430) utilisable avant l'activation du mécanisme d'expulsion de dose afin de permettre l'administration du volume de la suspension pharmaceutique à un sujet,
dans lequel le système de préparation de dose comprend en outre un élément d'agitation (40, 140, 240, 340, 440) apte à être animé d'un mouvement relatif d'agitation par rapport au réservoir à volume variable (10, 110, 210, 310, 410), le mouvement relatif d'agitation provoquant une agitation de remise en suspension de la suspension pharmaceutique, et
dans lequel l'élément d'agitation (40, 140, 240, 340, 440) est couplé de manière fonctionnelle à l'élément de préparation (30, 130, 230, 330, 430) et configuré pour subir ledit mouvement relatif d'agitation en réponse au fonctionnement de l'élément de préparation (30, 130, 230, 330, 430)
**caractérisé en ce que** le réservoir à volume variable (10, 110) est un réservoir souple en feuille et l'élément d'agitation (40, 140) comprend un élément de déformation (44a, 144a) adapté à déformer le réservoir souple en feuille pour provoquer ainsi l'agitation de remise en suspension de la suspension pharmaceutique, l'élément de déformation (44a, 144a) étant adapté à déformer le réservoir souple en feuille en balayant et en pressant une surface extérieure de celui-ci.

2. Dispositif de distribution de dose selon la revendication 1, dans lequel le mouvement relatif d'agitation comprend un mouvement de l'élément d'agitation (40, 140, 240, 340, 440) par rapport au réservoir à volume variable (10, 110, 210, 310, 410) depuis une première position prédéterminée jusqu'à une deuxième position prédéterminée.

3. Dispositif de distribution de dose selon la revendication 1 ou 2, comprenant en outre un verrou libérable (143, 460) commutable entre un état initial dans lequel l'activation du mécanisme d'expulsion de dose est empêchée et un état libéré dans lequel l'activation du mécanisme d'expulsion de dose est permise, le verrou libérable (143, 460) étant couplé fonctionnellement à l'élément de préparation (130, 430) et configuré pour commuter entre l'état initial et l'état libéré en réponse à la mise en fonctionnement de l'élément de préparation (130, 430).

4. Dispositif de distribution de dose selon l'une quelconque des revendications précédentes, comprenant en outre un logement (2, 3) accueillant au moins une portion du mécanisme d'expulsion de dose et définissant un axe de référence,
dans lequel l'élément de préparation (30) comprend un capuchon fixé de manière amovible au logement (2, 3) pour recouvrir la sortie (12),
dans lequel l'élément de déformation (44a) est fixé au capuchon ou fait partie de celui-ci, et
dans lequel le capuchon est adapté à être retiré par un mouvement axial relatif par rapport au logement (2, 3) et au réservoir souple en feuille, l'élément de déformation (44a) balayant et pressant ainsi la surface extérieure du réservoir souple en feuille.

5. Dispositif de distribution de dose selon la revendication 4, dans lequel l'élément d'agitation (40) comprend en outre un deuxième élément de déformation (44b) et un troisième élément de déformation (44c) agencés axialement espacés l'un de l'autre et de l'élément de déformation (44a), et
dans lequel au moins deux des éléments de déformation (44a, 44b, 44c) coupent l'axe de référence selon des angles différents.

6. Dispositif de distribution de dose selon l'une quelconque des revendications 1 à 3, comprenant en outre un logement (102, 103) accueillant au moins une portion du mécanisme d'expulsion de dose et définissant un axe de référence,
dans lequel l'élément de préparation (130) comprend une languette de traction fixée de manière amovible au logement (102, 103),
dans lequel l'élément de déformation (144a) est fixé à la languette de traction ou fait partie de celle-ci, et
dans lequel la languette de traction est adaptée à être retirée par un mouvement transversal relatif par rapport au logement (102, 103) et au réservoir souple en feuille, l'élément de déformation (144a) balayant et pressant ainsi la surface extérieure du réservoir souple en feuille.

7. Dispositif de distribution de dose selon la revendication 6, dans lequel l'élément d'agitation (140) comprend en outre un deuxième élément de déformation (144b) agencé transversalement espacé de l'élément de déformation (144a), et
dans lequel les deux éléments de déformation (144a, 144b) coupent l'axe de référence selon des angles différents.

8. Dispositif de distribution de dose selon la revendication 6 ou 7, comprenant en outre un capuchon (150) fixé de manière amovible au logement (102, 103) pour recouvrir la sortie (112),
dans lequel l'élément d'agitation (140) et le capuchon (150) comprennent des éléments de contact à interaction mutuelle (143, 152) configurés pour empêcher le retrait du capuchon (150) lorsque la languette de traction est fixée au logement (102, 103).

9. Dispositif de distribution de dose selon l'une quelconque des revendications 6 à 8, dans lequel le mécanisme d'expulsion de dose comprend un actionneur (120) et un élément de compression (127, 128) adapté à replier le réservoir souple en feuille en réponse à un déplacement axial de l'actionneur (120) par rapport au logement (102, 103) depuis une première position axiale jusqu'à une deuxième position axiale, et
dans lequel l'élément d'agitation (140) est configuré pour bloquer le mouvement de l'actionneur (120) depuis la première position axiale jusqu'à la deuxième position axiale lorsque la languette de traction est fixée au logement (102, 103).
